(19) **Europäisches Patentamt** / **European Patent Office** / **Office européen des brevets**

(11) **EP 4 380 968 B1**

(12) **EUROPEAN PATENT SPECIFICATION**

(45) Date of publication and mention
of the grant of the patent:
**01.10.2025 Bulletin 2025/40**

(21) Application number: **22761216.5**

(22) Date of filing: **05.08.2022**

(51) International Patent Classification (IPC):
*A61K 39/395* (2006.01)    *A61P 35/00* (2006.01)
*A61K 38/17* (2006.01)    *C07K 14/74* (2006.01)
*C07K 16/28* (2006.01)

(52) Cooperative Patent Classification (CPC):
(C-Sets available)
**A61P 35/00; A61K 38/1774; C07K 14/70539;**
**C07K 16/2818;** A61K 39/39558; C07K 2317/73;
C07K 2319/30    (Cont.)

(86) International application number:
**PCT/EP2022/072130**

(87) International publication number:
**WO 2023/012347 (09.02.2023 Gazette 2023/06)**

(54) **PHARMACEUTICAL COMPOSITIONS COMPRISING HLA FUSION PROTEINS**

PHARMAZEUTISCHE ZUSAMMENSETZUNGEN MIT HLA-FUSIONSPROTEINEN

COMPOSITIONS PHARMACEUTIQUES COMPRENANT DES PROTÉINES DE FUSION HLA

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **05.08.2021 EP 21190004**
**05.08.2021 EP 21190005**
**09.11.2021 EP 21207324**

(43) Date of publication of application:
**12.06.2024 Bulletin 2024/24**

(73) Proprietor: **ImmunOs Therapeutics AG**
**8952 Schlieren (CH)**

(72) Inventors:
• **MARROQUIN BELAUNZARAN, Osiris**
**8952 Schlieren (CH)**
• **RAFIEI, Anahita**
**8952 Schlieren (CH)**
• **KUMAR, Anil**
**8952 Schlieren (CH)**
• **RENNER, Christoph**
**8952 Schlieren (CH)**

(74) Representative: **Schulz Junghans**
**Patentanwälte PartGmbB**
**Großbeerenstraße 71**
**10963 Berlin (DE)**

(56) References cited:
**WO-A1-2016/124661    WO-A1-2017/153438**
**WO-A1-2018/029284**

• **AROSA ET AL: "Open conformers: the hidden**
**face of MHC-I molecules", TRENDS IN**
**IMMUNOLOGY, ELSEVIER LTD. TRENDS**
**JOURNALS, GB, vol. 28, no. 3, 26 February 2007**
**(2007-02-26), pages 115 - 123, XP005905268,**
**ISSN: 1471-4906, DOI: 10.1016/J.IT.2007.01.002**

Remarks:
The complete document including Reference
Table(s) and the Sequence Listing(s) can be
downloaded from the EPO website

(52) Cooperative Patent Classification (CPC): (Cont.)

C-Sets
**A61K 38/1774, A61K 2300/00;**
A61K 39/39558, A61K 2300/00

## Description

The present invention claims priority from the European applications EP21190004.8 and EP21190005.5 filed on 5 August 2021, and EP21207324.1 filed on 9 November 2021.

The present invention relates to pharmaceutical compositions for use as antineoplastic medicaments, said compositions comprising an HLA fusion protein associated with a beta-2-microglobulin polypeptide, and having desirable immunomodulatory and recombinant protein expression properties.

Background of the Invention

Classical MHC-I molecules (also known as HLA-I in humans) are dimeric or trimeric structures comprising a membrane-bound heavy chain characterized by an extracellular domain (comprising an $\alpha 1$, $\alpha 2$, and an $\alpha 3$ domain). The HLA heavy chain forms a complex with $\beta$2-microglobulin ($\beta$2m), also known as the HLA light chain, and presents antigen in form of a small peptide epitope associated with the HLA heavy chain peptide-binding cleft at the surface of the cell. MHC Class I molecules may also disassociate into free heavy chains lacking $\beta$2m, and/or peptide epitope (Arosa et al. Trends in Immunology 2007 Mar; 28(3):115-23). The inventors have identified selected HLA, such as HLA-B27, or the HLA-B57 haplotype linked to certain immune correlates in HIV infection, as promising immunomodulatory medicaments when delivered as a fusion protein with a stabilizing peptide such as an immunoglobulin (Ig) Fc portion, particularly where the HLA lacks association with $\beta$2m, or a peptide epitope in the HLA binding cleft (see WO 2017153438 A1, WO2016124661A1, WO2018 029284 A1).

To obtain isolated non-$\beta$2m-associated HLA used in previous applications of this technology, the HLA heavy chain fusion protein / $\beta$2m complex is separated, for example under acidic conditions, prior to purification, for example by size-based chromatography, prior to refolding of the isolated HLA heavy chain fusion proteins. However, while non-$\beta$2m-associated HLA heavy chain compounds, such as the HLA-B57 heavy chain-derived fusion protein studied herein, have desirable immunomodulatory qualities, upscaling of manufacturing to industrial quantities has revealed challenges. Up to half of the HLA fusion protein is lost during the process of separating the immunomodulatory HLA fusion protein from the $\beta$2m.

Based on the above-mentioned state of the art, the objective of the present invention is to provide an improved pharmaceutical compositions an HLA heavy chain-based fusion proteins with favorable immunomodulatory properties, for use in treating malignant neoplastic diseases. This objective is attained by the subject-matter of the independent claims of the present specification, with further advantageous embodiments described in the dependent claims, examples, figures and general description of this specification.

Summary of the Invention

By investigating the immunological qualities of an intermediate, HLA heavy chain fusion protein / $\beta$2m polypeptide complex in comparison to an HLA heavy chain fusion protein lacking $\beta$2m polypeptide, the inventors made the surprising observation that the HLA fusion protein / $\beta$2m polypeptide complex molecule exhibits desirable immunomodulatory properties, and delivers an increased beneficial therapeutic effect in treating cancer, particularly in a humanized immune system model of cancer.

A first aspect of the invention relates to pharmaceutical compositions for use in treating malignant neoplastic disease, said composition comprising an HLA fusion protein (comprising an MHC class I HLA heavy chain extracellular domain polypeptide, and a stabilizing immunoglobulin (Ig) fragment crystallizable (Fc) polypeptide), where the HLA fusion protein is additionally associated with a $\beta$2m polypeptide, and where the compositions contain at least one pharmaceutically acceptable carrier, diluent or excipient. In particular embodiments, the HLA heavy chain portion of the HLA fusion protein is the extracellular domain of a naturally-occurring HLA heavy chain selected from HLA-B57, HLA-C08, HLA-A25, HLA-B58, HLA-B27, HLA-A30, HLA-B53, or HLA-C12. In other particular embodiments, the HLA fusion protein comprises a variant HLA heavy chain polypeptide, particularly a variant of the HLA-B57 heavy chain, where the variant has at least 95% similarity to one of the HLA heavy chains listed above, and retains a similar biological function.

Further embodiments of the pharmaceutical composition for use according to the invention relate to non-covalent association of the HLA fusion protein with the $\beta$2m polypeptide, and variant HLA-B57 heavy chain polypeptides, Ig Fc polypeptides, peptide linkers, and secretion signals of particular use for inclusion in such pharmaceutical compositions, as well as combination medicaments further comprising a checkpoint inhibition agent, wherein said checkpoint inhibitory agent is an antibody capable of binding to one of CTLA-4, PD-1, PD-L1, or PD-L2 with a dissociation constant of $10^{-7}$ mol/L or lower.

Another aspect of the invention relates to checkpoint inhibitor agents for use in treating a patient with cancer, especially a blood cancer, or a malignant neoplastic disease, formulated for administration in combination with a pharmaceutical composition comprising an HLA fusion protein and $\beta$2m according to the first aspect of the invention, wherein said checkpoint inhibitory agent is an antibody capable of binding to one of CTLA-4, PD-1, PD-L1, or PD-L2 with a dissociation constant of $10^{-7}$ mol/L or lower.

*Terms and definitions*

For purposes of interpreting this specification, the following definitions will apply and whenever appropriate, terms used in the singular will also include the plural and vice versa. In the event that any definition set forth below conflicts with any document referenced herein, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *polypeptide* in the context of the present specification relates to a molecule consisting of 50 or more amino acids that form a linear chain wherein the amino acids are connected by peptide bonds. The amino acid sequence of a polypeptide may represent the amino acid sequence of a whole (as found physiologically) protein or fragments thereof. The term "polypeptides" and "protein" are used interchangeably herein and include proteins and fragments thereof. Polypeptides are disclosed herein as amino acid residue sequences.

Amino acid residue sequences are given from amino to carboxyl terminus. Capital letters for sequence positions refer to L-amino acids in the one-letter code (Stryer, Biochemistry, 3rd ed. p. 21). Lower case letters for amino acid sequence positions refer to the corresponding D- or (2R)-amino acids. Sequences are written left to right in the direction from the amino to the carboxy terminus. In accordance with standard nomenclature, amino acid residue sequences are denominated by either a three letter or a single letter code as follows: Alanine (Ala, A), Arginine (Arg, R), Asparagine (Asn, N), Aspartic Acid (Asp, D), Cysteine (Cys, C), Glutamine (Gln, Q), Glutamic Acid (Glu, E), Glycine (Gly, G), Histidine (His, H), Isoleucine (Ile, I), Leucine (Leu, L), Lysine (Lys, K), Methionine (Met, M), Phenylalanine (Phe, F), Proline (Pro, P), Serine (Ser, S), Threonine (Thr, T), Tryptophan (Trp, W), Tyrosine (Tyr, Y), and Valine (Val, V).

The terms *gene expression* or *expression,* or alternatively the term *gene product,* may refer to either of, or both of, the processes - and products thereof - of generation of nucleic acids (RNA) or the generation of a peptide or polypeptide, also referred to transcription and translation, respectively, or any of the intermediate processes that regulate the processing of genetic information to yield polypeptide products. The term *gene expression* may also be applied to the transcription and processing of an RNA gene product, for example a regulatory RNA or a structural (e.g. ribosomal) RNA. If an expressed polynucleotide is derived from genomic DNA, expression may include splicing of the mRNA in a eukaryotic cell. Expression may be assayed both on the level of transcription and translation, in other words mRNA and/or protein product.

In the context of the present specification, the terms *sequence identity, sequence similarity* and *percentage of sequence identity* refer to a single quantitative parameter representing the result of a sequence comparison determined by comparing two aligned polypeptide sequences position by position. Methods for alignment of sequences for comparison are well-known in the art. Alignment of sequences for comparison may be conducted by the local homology algorithm of Smith and Waterman, Adv. Appl. Math. 2:482 (1981), by the global alignment algorithm of Needleman and Wunsch, J. Mol. Biol. 48:443 (1970), by the search for similarity method of Pearson and Lipman, Proc. Nat. Acad. Sci. 85:2444 (1988) or by computerized implementations of these algorithms, including, but not limited to: CLUSTAL, GAP, BESTFIT, BLAST, FASTA and TFASTA. Software for performing BLAST analyses is publicly available, e.g., through the National Center for Biotechnology-Information (http://blast.ncbi.nlm.nih.gov/).

One example for comparison of amino acid sequences is the BLASTP algorithm that uses the default settings: Expect threshold: 10; Word size: 3; Max matches in a query range: 0; Matrix: BLOSUM62; Gap Costs: Existence 11, Extension 1; Compositional adjustments: Conditional compositional score matrix adjustment. Unless stated otherwise, sequence identity values provided herein refer to the value obtained using the BLAST suite of programs (Altschul et al., J. Mol. Biol. 215:403-410 (1990)) using the above identified default parameters for protein.

Reference to identical sequences without specification of a percentage value implies 100% identical sequences (i.e. the same sequence).

In the context of the present specification, the term *dissociation constant ($K_D$)* is used in its meaning known in the art of chemistry and physics; it refers to an equilibrium constant that measures the propensity of a complex composed of [mostly two] different components to dissociate reversibly into its constituent components. The complex can be e.g. an antibody-antigen complex AbAg composed of antibody Ab and antigen Ag. $K_D$ is expressed in molar concentration [mol/L] and corresponds to the concentration of [Ab] at which half of the binding sites of [Ag] are occupied, in other words, the concentration of unbound [Ab] equals the concentration of the [AbAg] complex. The dissociation constant can be calculated according to the following formula:

$$K_D = \frac{[Ab] * [Ag]}{[AbAg]}$$

[Ab]: concentration of antibody; [Ag]: concentration of antigen; [AbAg]: concentration of antibody-antigen complex

As used herein, the term *pharmaceutical composition* refers to an HLA fusion protein associated with β2m according to the invention, together with at least one pharmaceutically acceptable carrier. In certain embodiments, the pharmaceutical composition according to the invention is provided in a form suitable for topical, parenteral or injectable administration.

As used herein, the term *pharmaceutically acceptable carrier* includes any solvents, dispersion media, coatings, surfactants, antioxidants, preservatives (for example, antibacterial agents, antifungal agents), isotonic agents, absorption delaying agents, salts, preservatives, drugs, drug stabilizers, binders, excipients, disintegration agents, lubricants, sweetening agents, flavoring agents, dyes, and the like and combinations thereof, as would be known to those skilled in the art (see, for example, Remington: the Science and Practice of Pharmacy, ISBN 0857110624).

As used herein, the term *treating* or *treatment* of any disease or disorder (e.g. cancer) refers in one embodiment, to ameliorating the disease or disorder (e.g. slowing or arresting or reducing the development of the disease, or at least one of the clinical symptoms thereof, for example, slowing, or reducing tumor growth). In another embodiment "treating" or "treatment" refers to alleviating or ameliorating at least one physical parameter including those which may not be discernible by the patient. In yet another embodiment, "treating" or "treatment" refers to modulating the disease or disorder, either physically, (e.g., stabilization of a discernible symptom), physiologically, (e.g., stabilization of a physical parameter), or both. Methods for assessing treatment and/or prevention of disease are generally known in the art, unless specifically described hereinbelow.

In the context of the present specification, the term *peptide linker,* or *amino acid linker* refers to a polypeptide of variable length that is used to connect two polypeptides in order to generate a single chain polypeptide. Exemplary embodiments of linkers useful for practicing the invention specified herein are oligopeptide chains consisting of 1, 2, 3, 4, 5, 10, 20, 30, 40 or 50 amino acids. A nonlimiting example of an amino acid linker is the polypeptide GGGGSGGGGS (SEQ ID NO 003) that links an HLA heavy chain polypeptide with a stabilizing peptide, for example, linking an HLA-B57 polypeptide with an IgG4 Fc polypeptide in an HLA fusion protein.

The term *Human leukocyte antigen (HLA) heavy chain, HLA heavy chain* in the context of the present specification relates to the protein encoded by an *MHC Class I histocompatibility antigen* gene, particularly a classical, MHC class 1a heavy chain. In humans, an HLA heavy chain can be a monomer, or form a part of dimeric structures comprising a heavy chain with three extracellular domains (α1, α2, and α3), bound non-covalently to a β2m light chain, or optionally, trimeric structures wherein a small peptide is associated at the peptide-binding cleft. Full length *HLA heavy chain* polypeptides comprise an extracellular domain comprising an α1, an α2, and an α3 domain, a transmembrane domain, and an intracellular domain.

A list of naturally occurring *HLA heavy chains* which are considered embodiments of the term according to this aspect of the invention are listed in Table 1.

**Table 1: List of HLA heavy chain alleles**

| HLA-A | HLA-B | | HLA-C |
|---|---|---|---|
| A01 | B07 | B53 | C01 |
| A02 | B08 | B54 | C02 |
| A03 | B13 | B55 | C03 |
| A11 | B14 | B56 | C04 |
| A23 | B15 | B57 | C05 |
| A24 | B18 | B58 | C06 |
| A25 | B27 | B59 | C07 |
| A26 | B35 | B67 | C08 |
| A29 | B37 | B73 | C12 |
| A30 | B38 | B78 | C14 |
| A31 | B39 | B81 | C15 |
| A32 | B40 | B82 | C16 |
| A33 | B42 | B83 | C17 |
| A34 | B44 | | C18 |
| A36 | B46 | | |
| A43 | B47 | | |
| A66 | B48 | | |
| A68 | B49 | | |
| A69 | B50 | | |
| A74 | B51 | | |
| A80 | B52 | | |

The term *variant* in the context of the present specification relates an HLA heavy chain polypeptide sequence with at least one amino acid residue that differ from a naturally-occurring polypeptide sequence. For example, a variant HLA heavy chain polypeptide in which one, or several amino acid substitutions have been introduced, such that it differs from the original, naturally occurring human protein sequence it is derived from.

The term *extracellular domain* as applied to an HLA heavy chain, or a variant of an HLA heavy chain in the context of this specification, refers to the extracellular portion of an HLA heavy chain polypeptide which extends from the cell surface. The extracellular portion of an HLA Class 1a polypeptide comprises the alpha ($\alpha$) 1 domain, $\alpha$2 domain, and $\alpha$3 domain, which comprise regions essential for receptor ligand interactions which mediate the immunomodulatory effects of the HLA fusion protein in the pharmaceutical composition for use according to the invention. The *extracellular domain* excludes the transmembrane domain, and the intracellular domain.

In the context of the present specification, the term *HLA fusion protein* refers to a recombinant polypeptide which comprises the extracellular domain of an HLA heavy chain, joined to a stabilizing immunoglobulin (Ig) Fc, optionally by means of a peptide linker. The term encompasses such an *HLA fusion protein* in complex with a β2-microglobulin polypeptide as secreted from mammalian cell culture, as well as purified *HLA fusion protein* which is not associated with β2-microglobulin. The term *HLA fusion protein* may refer to a monomer comprising a single HLA polypeptide joined to a single stabilizing immunoglobulin (Ig) Fc domain, or a dimer formed by association of a first HLA fusion protein monomer, and a second HLA fusion protein monomer, particularly joined via their Ig Fc domains.

In the context of the present specification, the term β2-microglobulin (*β2m, B2m), B2m polypeptide,* or *β2m polypeptide* refers to the beta (β) chain, also known as the HLA light chain of MHC class I molecules. The term β2-microglobulin encompasses firstly, a pre-processing β2-microglobulin comprising a secretory signal, for example, the sequence of Uniprot P61769, or the sequence SEQ ID NO 006, and secondly, the post-secretion form of the protein, in which a secretory signal portion of the protein has been removed by cleavage during the secretion process, as found in an HLA fusion protein : β2m polypeptide complex comprised within the pharmaceutical composition according to the invention.

In the context of the present specification, the term *secretory signal, secretory signal peptide* or *signal sequence* refers to an N-terminal leader sequence initiating the open reading frame (ORF) of a polypeptide, usually about 6-30 amino acids in length. In rare cases, a *secretory signal* is placed at the C-terminus of a polypeptide. *Secretory signals* are sometime referred to as targeting signals, localization signals, transit peptides, leader sequences, or leader peptides.

*Secretory signals* which enable efficient secretion of a polypeptide from cells are well known, and may be included in the ORF of a recombinant protein in order to facilitate export of a polypeptide to the supernatant in cell-based polypeptide manufacturing system, allowing purification of a polypeptide from the cell supernatant. Upon translation of the mRNA encoding the *secretory signal,* it is recognized by a cytosolic protein mediating transfer of the mRNA-ribosome complex to a channel protein in the endoplasmic reticulum (ER). The newly synthesized polypeptide comprising the *secretory signal peptide* is translocated to the ER lumen through the channel protein, entering the cell secretion pathway. *Signal sequences* of particular use according to the invention are those that are cleaved from the final polypeptide product following translation, for example, SEQ ID NO 004.

In the context of the present specification, the terms *immunoglobulin crystallizable fragment (Fc) region,* or *Ig Fc* refers to a fraction of an antibody, or immunoglobulin (Ig), comprised of a $C_H2$ and a $C_H3$ domain. *Ig Fc* encompass both a monomer, or a dimer comprising two *Ig Fc,* covalently linked by disulfide bonds. In the context of the HLA fusion protein according to the invention, disulfide bonds can join two HLA fusion proteins molecules, each comprising *Ig Fc* domains. The presence of the *Ig Fc* in the HLA fusion protein facilitates increased solubility, stability, avidity, half-life, and from a technological point of view, cost-effective production and purification in mammalian systems (protein A or G purification).

In the context of the present specification, the term *checkpoint inhibitory agent* encompasses antibodies capable of binding to one of CTLA-4, PD-1, PD-L1, or PD-L2 with a dissociation constant of $10^{-7}$ mol/L or lower, capable of disrupting an inhibitory signalling cascade that limits immune cell activation, and in particular T cell activation, known in the art as an immune checkpoint mechanism. Examples of *checkpoint inhibitory agent* include, for example, an antibody which binds specifically to CTLA-4 (Uniprot P16410), PD-1 (Uniprot Q15116), or PD-L1 (Uniprot Q9NZQ7).

The terms "cancer" and "malignant neoplastic disease" are used synonymously herein. Particular alternatives of any of the aspects and embodiments disclosed herein are directed at the use of the combinations of the invention in treatment of solid tumours. Other alternatives of any of the aspects and embodiments disclosed herein are directed at the use of the combinations of the invention in treatment of liquid cancers such as myelogenous or granulocytic leukemia, particularly AML, lymphatic, lymphocytic, or lymphoblastic leukemia and lymphoma, polycythemia vera or erythremia.

Detailed Description of the Invention

*Pharmaceutical composition comprising HLA fusion protein for treating cancer*

A first aspect of the invention relates to a pharmaceutical composition for use in treating a malignant, neoplastic disease, said composition comprising an HLA fusion protein, associated with a B2m polypeptide. Said HLA fusion protein according to the invention comprises

- a first polypeptide comprising the extracellular domain of an HLA heavy chain (particularly, including the extracellular $\alpha$1, $\alpha$2 and $\alpha$3 domains), and
- a second stabilizing polypeptide, namely the Ig Fc portion of an immunoglobulin.

Using the $\beta$2m-associated HLA fusion protein confers the advantage of increased yields of the immunomodulatory HLA fusion protein, as producing this product does not incur the loss of protein that occurs during a $\beta$2m disassociation step (Fig. 2), and enhances certain immunological properties such as phagocyte uptake of tumor cells, in comparison to a non-$\beta$2m-associated HLA fusion protein alternative (Fig 4). Importantly, the pharmaceutical composition according to the invention comprising an HLA fusion protein and $\beta$2m is associated with enhanced survival when using a pharmaceutical composition according to the invention to treat cancer in humanized mice comprising human immune cells, compared to an HLA fusion protein format lacking $\beta$2m polypeptide (Fig 5).

In some embodiments of the pharmaceutical composition for use according to the invention, the $\beta$2m polypeptide is linked by a peptide linker to the HLA fusion protein.

In particular embodiments of the pharmaceutical composition for use according to the invention, the $\beta$2m polypeptide is non-covalently associated with said HLA fusion protein.

In particular embodiments of the pharmaceutical composition for use according to the invention, the HLA fusion protein is associated with a $\beta$2m molecule at a molar ratio of between 3:5 to 7:5, more particularly between 4:5 to 6:5. In other words, the HLA fusion protein and the $\beta$2m polypeptide are present at a ratio of, or close to, 1 to 1. References to HLA to ß2M are to be understood as molar throughout this document, unless stated explicitly otherwise.

In particular embodiments of the pharmaceutical composition for use according to the invention, two HLA fusion proteins, each associated with a $\beta$2m polypeptide may be associated in a dimerized form via their Fc portions.

*HLA heavy chain polypeptides*

Certain domains of HLA heavy chain proteins not required for cognate ligand interactions, are not included in the HLA polypeptide portion of the HLA fusion protein comprised in the pharmaceutical composition for use according to the invention. The intracellular domain, and the transmembrane domain are absent from the HLA heavy chain polypeptide.

In particular embodiments of the methods to produce an HLA fusion protein, or the isolated HLA fusion protein according to the invention, the variant HLA-B57 polypeptide comprised in the HLA fusion protein includes the alpha 1, 2 and 3 domains of the naturally occurring HLA heavy chain protein, regions of which are essential for receptor ligand interactions which mediate the immunomodulatory effects of an HLA fusion protein according to the invention.

In more particular embodiments of the method to produce an HLA fusion protein, or the isolated HLA fusion protein according to the invention, the variant HLA-B57 polypeptide protein is the alpha 1, 2 and 3 domains of the naturally occurring HLA heavy chain protein, excepting the C-terminal isoleucine-valine dipeptide, preceded by the threonine-valine-proline residues of the extracellular domain, within the HLA-B57 region preceding the transmembrane domain sometimes annotated, or referred to, as the "connecting peptide".

Structural data suggests that HLA heavy chains interact with ligands such as Killer immunoglobulin-like receptors (KIR) and leukocyte immunoglobulin-like receptors (LILR) via regions distant from the transmembrane region. Amino acids close to the membrane do not generally interact with receptors. Furthermore, the inventors surmise that the high content of hydrophobic amino acids within the 5 C-terminal amino acid motif of the extracellular domain connecting peptide of naturally occurring HLA heavy chain sequences, is likely to introduce undesirable properties into recombinant proteins, such as a tendency towards protein aggregation, which can then affect the production, purification, stability and toxicity in downstream production processes.

In particular of the pharmaceutical composition for use according to the invention, the HLA heavy chain extracellular domain polypeptide of the HLA fusion protein component comprises the core structure of the extracellular portion of an HLA heavy chain protein sequence, comprising the alpha 1, 2, and 3 domains, as this portion confers the HLA fusion protein with the ability to interact with surface molecules on target cells.

In some embodiments of the pharmaceutical composition for use according to the invention, the HLA heavy chain polypeptide portion of the HLA fusion protein has the polypeptide sequence of the extracellular domain of a naturally occurring HLA heavy chain listed in Table. 1. In particular embodiments, the HLA heavy chain extracellular domain is that of a naturally occurring HLA heavy chain with immunomodulatory qualities, such as specific binding to regulatory KIR3DL1, LILRA and LILRB1/2 cell surface proteins which alter innate and adaptive immune cell function. In some such embodiments, the HLA heavy chain portion of the HLA fusion protein is derived from, or essentially is, the extracellular domain of HLA-B58. In some such embodiments, the HLA heavy chain portion of the HLA fusion protein is derived from, or essentially is, the extracellular domain of HLA-B27. In other embodiments, the HLA heavy chain portion of the HLA fusion protein is derived from, or essentially is, the extracellular domain of HLA-B44. In further embodiments, the HLA heavy chain portion of the HLA fusion protein is derived from, or essentially is, the extracellular domain of HLA-B81. In other embodiments, the HLA heavy chain portion of the HLA fusion protein is derived from, or essentially is, the extracellular domain of HLA-C12. In particular embodiments, the HLA heavy chain portion of the HLA fusion protein is derived from, or essentially is, the extracellular domain of HLA-B57.

In another embodiment of the pharmaceutical composition according to the invention, the HLA fusion protein comprises the extracellular domain of an HLA-Cw08:02 heavy chain characterized by an E at position 46, and an R at position 97, as the inventors found this polypeptide to have favorable expression qualities

In further embodiments of the pharmaceutical composition for use in the treatment of cancer, said composition comprises:

a. an HLA fusion protein comprising:

i. an extracellular domain of an HLA-C08 heavy chain characterized by an E at position 46, and an R at position 97; or a variant of the extracellular domain of an HLA-C08 heavy chain, wherein said variant is characterized by a sequence similarity of at least ($\geq$) 95%, particularly $\geq$98%, and a similar biological activity;
ii. an immunoglobulin crystallizable fragment (Ig Fc) polypeptide, particularly an IgG Fc polypeptide, more particularly an IgG4 Fc polypeptide; and

b. a $\beta$2m polypeptide;

particularly wherein the HLA fusion protein comprises the polypeptide SEQ ID NO 010, more particularly wherein the HLA fusion protein consists of the sequence SEQ ID NO 010.

In another embodiment of the pharmaceutical composition according to the invention, the HLA fusion protein comprises the extracellular domain of an HLA-B58:01 heavy chain characterized by an E at position 46, and an R at position 97, as the inventors found this heavy chain to have favorable expression qualities.

In further embodiments of the pharmaceutical composition for use in the treatment of cancer, said composition comprises:

a. an HLA fusion protein comprising:

i. an extracellular domain of an HLA-B58 heavy chain characterized by an E at position 46, and an R at position 97; or a variant of the extracellular domain of an HLA-B58 heavy chain, wherein said variant is characterized by a sequence similarity of at least (≥) 95%, particularly ≥98%, and a similar biological activity;
ii. an immunoglobulin crystallizable fragment (Ig Fc) polypeptide, particularly an IgG Fc polypeptide, more particularly an IgG4 Fc polypeptide; and

b. a β2m polypeptide;

particularly wherein the HLA fusion protein comprises the polypeptide SEQ ID NO 009, more particularly wherein the HLA fusion protein consists of the sequence SEQ ID NO 009. In particular embodiments of the pharmaceutical composition for use according to the invention, the HLA fusion protein comprises a variant HLA heavy chain extracellular domain polypeptide. Said variant HLA heavy chain polypeptide is characterized by a sequence similarity (on the protein level) of at least (≥) 95% to the non-variant extracellular domain of an HLA heavy chain, and having a similar biological activity compared to the original sequence in the context of an HLA fusion protein. In particular embodiments, the variant HLA heavy chain is ≥98% similar to the naturally occurring HLA heavy chain extracellular domain from which it is derived, while having a similar biological activity compared to the original sequence in the context of an HLA fusion protein.

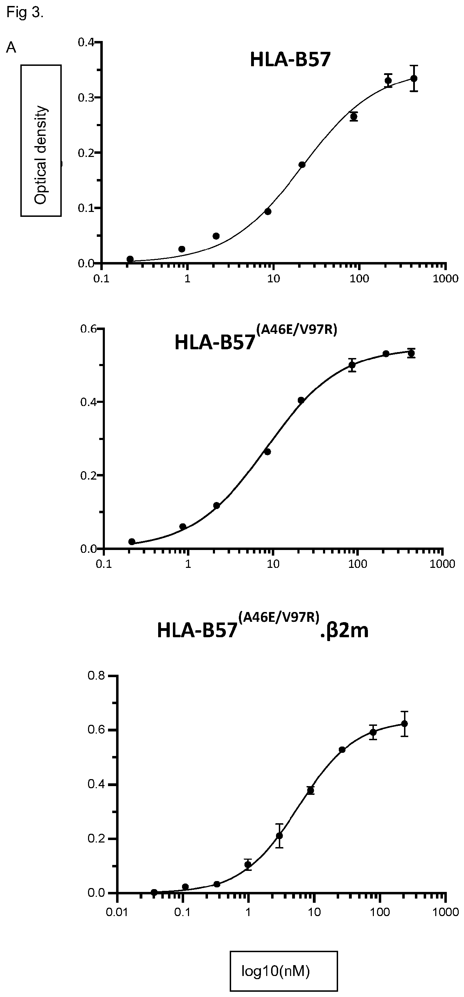

A "similar biological activity" is defined as at least 65%, or particular 85%, or over 100% of the capacity of a variant HLA heavy chain polypeptide HLA fusion protein to bind to LILRB2, compared to the equivalent non-variant structure, as measured by an enzyme-linked immunosorbent assay (ELISA) method. Biological activity may be assessed by calculating the EC50, i.e. the concentration of a fusion protein that gives a half-maximal response, in this case, half the maximal binding to a biotinylated LILRB2 molecule. For example, the EC50 of equivalent non-β2m-associated HLA-B57 and HLA-B57$^{(A46E/V97R}$ heavy chain fusion proteins is shown to be 21nM, and 8.3nM respectively, demonstrating the biological function of the variant exceeds that of the wildtype sequence (Fig. 3A).

To determine the EC50 according to the invention, streptavidin coated high binding capacity 96 well plates may be coated with 50 μl of c-terminally biotinylated LILRB2 (for example, obtained from BPS Bioscience #100335) at a final concentration of 5 μg/ml in PBS buffer. PBS and IgG isotype may be used as negative controls. A serial dilution of HLA fusion protein is applied in a titrated series, (for example, eight concentration points: 10, 2.5, 1, 0.25, 0.1, 0.025, 0.01, 0.0025 μg/ml), preferably applied in 50ul duplicates. A labelled antibody that can detect the fusion protein (for example, an APC conjugated goat anti-human IgG antibody (Jackson Immuno Research #109-135-098, 1:100 dilution in TBS 50 μl) may then be applied to detect HLA fusion protein binding and background, and fluorescence excitation and emission measured at the appropriate wavelength (for example 650 nm & 660 nm). A three-parameter based log (agonist) model is one suitable means to determine the EC50 of the HLA fusion protein binding to the LILRB2 ELISA.

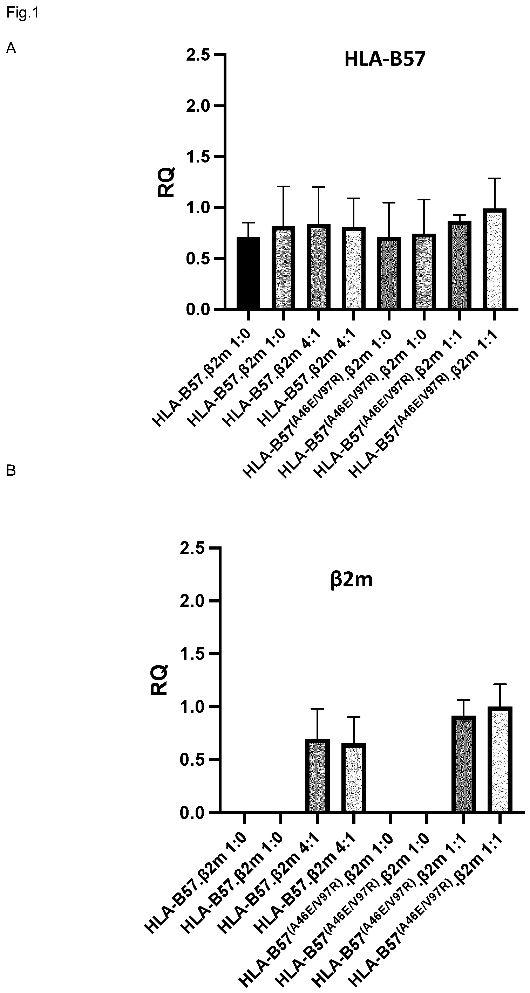

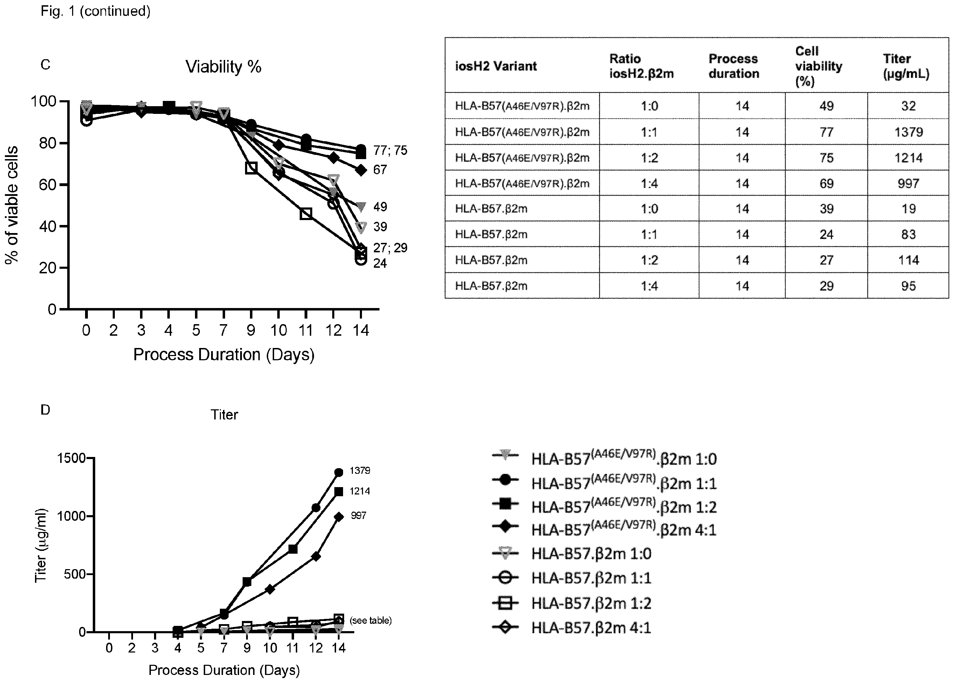

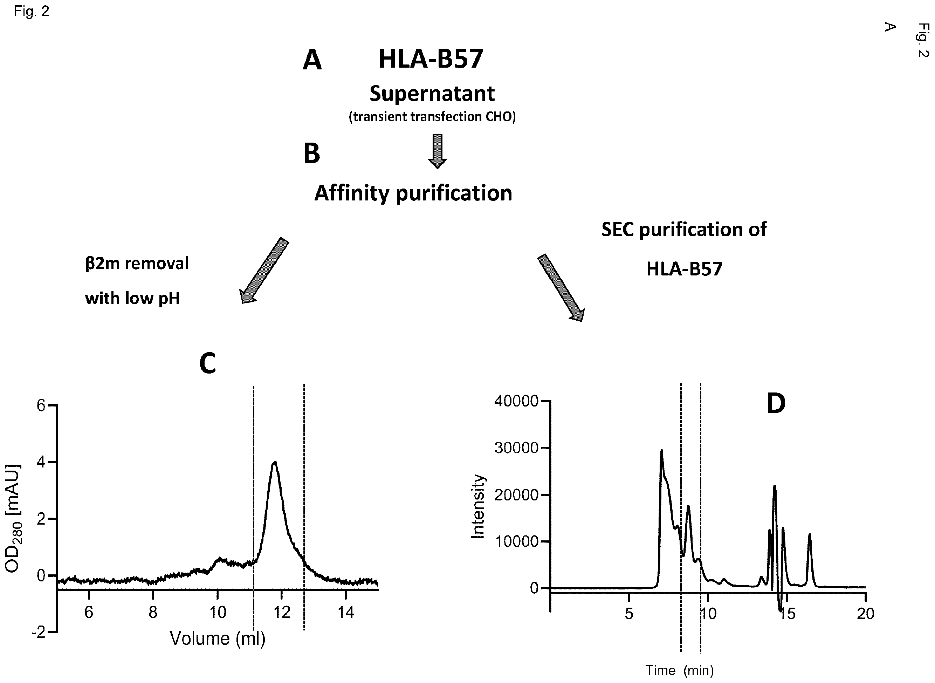

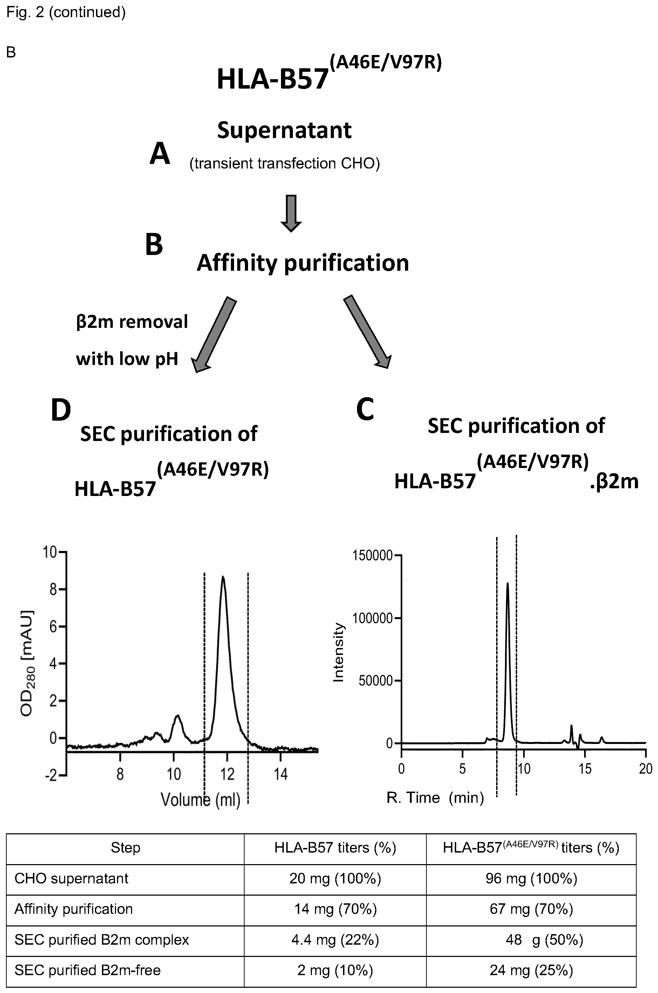

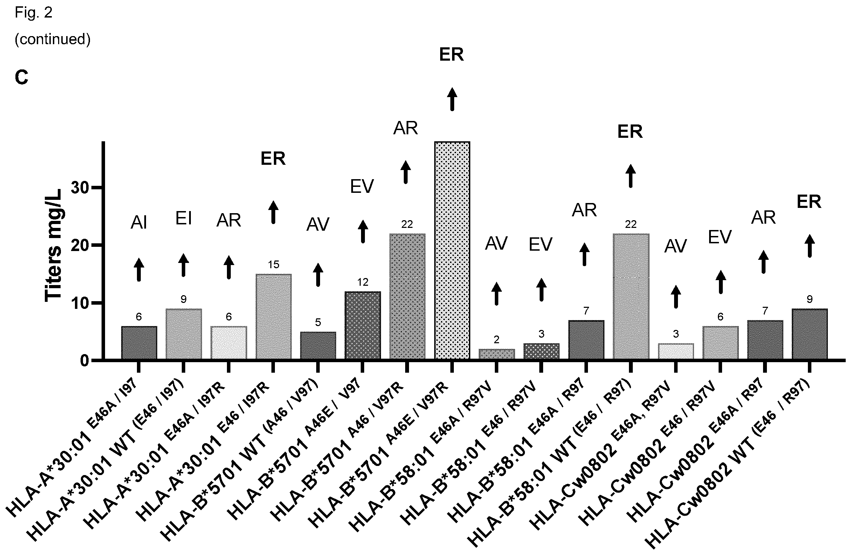

In particular embodiments of the pharmaceutical composition for use according to the invention, HLA fusion protein comprises a variant HLA-B57 heavy chain extracellular domain polypeptide, which is characterized by at least one, or two specific amino acid substitutions which differ from the polypeptide sequence a naturally-occurring HLA heavy chain extracellular domain polypeptide. The HLA-B57 heavy chain gene family currently encompasses 221 known variants with unique nucleic acid sequences, numbering HLA-B*57:01 to HLA-B*57:141, encoding several dozen unique protein sequences. The protein sequence for which are known, and may be retrieved, for example, by entering the search term "B*57" into the MGT/HLA Allele Query Form provided by the European Bioinformatics Institute Immuno Polymorphism Database, Robinson J. *et al.* 2013 *Nucleic Acids Res.* 41:D1234, https://www.ebi.ac.uk/ipd/imgt/hla/allele.html). In more particular embodiments, the HLA heavy chain polypeptide is a variant of the naturally occurring extracellular domain of an HLA-B57 heavy-chain polypeptide sequence, in which one, or two amino acid substitutions have been performed such that the variant HLA heavy chain polypeptide is characterized by an E at position 46, and an R at position 97. In other words, an amino acid other than E has been replaced with an E at position 46, and/or an amino acid other than R, has been replaced with an R at position 97. The numbering of these amino acids refers to the assignment of integers sequentially beginning with the G, S, H motif (or equivalent motif, for example, C, S, H in the HLA-C08 polypeptides) that initiates the extracellular domain of a secreted HLA-B57 portion, lacking the secretion signal, with the numbers 1, 2, and 3. The inventors find these amino acid substitutions correlate with high yields of the resulting variant-based HLA fusion protein compared to the wildtype sequence (Fig. 1, and Fig. 2), and a favorable LILRB2 binding profile.

In more particular embodiments of the pharmaceutical composition for use according to the invention, the HLA heavy chain extracellular polypeptide portion of the HLA fusion protein according to the invention comprises the variant HLA-B57 sequence designated SEQ ID NO 001. In still more particular embodiments, the HLA heavy chain polypeptide essentially consists of the sequence designated SEQ ID NO 001.

In another embodiment of the pharmaceutical composition according to the invention, the HLA fusion protein comprises the variant of an extracellular domain of an HLA-A30:01 heavy chain characterized by an E at position 46, and an R at position 97, as the inventors found this heavy chain to have favorable expression qualities.

In further embodiments of the pharmaceutical composition for use in the treatment of cancer, said composition comprises:

> a. an HLA fusion protein comprising:

>> iii. an extracellular domain of an HLA-A30 heavy chain characterized by an E at position 46, and an R at position 97; or a variant of the extracellular domain of an HLA-A30 heavy chain, wherein said variant is characterized by a sequence similarity of at least ($\geq$) 95%, particularly $\geq$98%, and a similar biological activity;
>> iv. an immunoglobulin crystallizable fragment (Ig Fc) polypeptide, particularly an IgG Fc polypeptide, more particularly an IgG4 Fc polypeptide; and

> b. a $\beta$2m polypeptide;

particularly wherein the HLA fusion protein comprises the polypeptide SEQ ID NO 012, more particularly wherein the HLA fusion protein consists of the sequence SEQ ID NO 012.

In certain embodiments of the pharmaceutical composition for use according to the invention, the HLA heavy chain polypeptide and the Ig Fc polypeptide are joined by a peptide linker. In particular embodiments, the peptide linker is between 5 and 20 amino acids in length. In more particular embodiments this joining peptide linker has the sequence SEQ ID NO 003.

In particular embodiments of the pharmaceutical composition for use according to the invention, the HLA fusion protein comprises a polypeptide with the sequence designated SEQ ID NO 005, and is associated with a $\beta$2m polypeptide. In other embodiments, the HLA fusion protein additionally comprises a secretory signal upstream of the sequence SEQ ID NO 005. In particular embodiments, the secretory signal upstream of the HLA fusion protein polypeptide is SEQ ID NO 004. In other embodiments, the HLA fusion protein essentially consists of a secretory signal of SEQ ID NO 004, joined to a polypeptide designated SEQ ID NO 005. In more particular embodiments of the pharmaceutical composition for use according to the invention, the HLA fusion protein essentially consists of the sequence designated SEQ ID NO 005 (comprising a variant extracellular domain of HLA-B57 fused to an IgG4 Fc), and is associated with a $\beta$2m polypeptide.

In particular embodiments of the pharmaceutical composition for use according to the invention, the $\beta$2m polypeptide associated with the HLA fusion protein has the sequence designated SEQ ID NO 006.

In alternative embodiments of the pharmaceutical composition for use according to the invention, the HLA fusion protein comprises an HLA-B57 heavy chain polypeptide identical to SEQ ID NO 001, other than a single differing amino acid residue (wherein the single differing amino acid residue is not position 46, or 97). In other words, in addition to the one, or in some cases, two amino acid substitutions at positions 46, and/or 97, the recombinant HLA-B57 polypeptide according to this embodiment includes at least one further amino acid substitution compared to the naturally occurring HLA-B57 molecule from which it is derived.

*Stabilizing and linking peptides*

The HLA fusion portion of the pharmaceutical composition for use according to the invention, comprises a stabilizing polypeptide conferring stability during expression and purification. The presence of stabilizing portion of the HLA fusion protein increases the yield and solubility by reducing degradation and oligomerization of the HLA fusion protein, and further associated with improved viability of cells expressing the fusion protein.

The stabilizing polypeptide of the HLA fusion protein according to the invention is a human Ig Fc polypeptide. An Ig Fc portion may also prolong the in vivo half-life of a molecule *in vivo* by binding to recycling receptors. In particular embodiments of the pharmaceutical composition for use according to the invention, the stabilizing polypeptide is an isotype IgG Fc. An IgG Fc stabilizing peptide domain delivers an added advantage during purification of the HLA fusion protein, by enabling absorption to a protein A or G coated surface.

In particular embodiments of the pharmaceutical composition for use according to the invention, the HLA fusion protein comprises an IgG4 polypeptide, which is desirable isotype in therapeutic fusion proteins due to its low cytotoxicity. In still more particular embodiments of the pharmaceutical composition for use according to the invention, the HLA fusion protein comprises an altered IgG4 S228P.dk molecule with sequence SEQ ID NO 002. This is characterized by a mutation in the hinge region of the IgG4, where Proline (P) is substituted for serine (S) at position 228 of the original IgG4 antibody, and dK indicates a deletion of the last amino acid Lysine (K) on the original IgG4 sequence. These changes give the IgG4 format stability and less heterogenicity. Both changes are well established and used commonly in diverse Fc constructs.

In certain embodiments of the pharmaceutical composition for use according to the invention, the HLA fusion protein comprises an HLA polypeptide joined with an IgG polypeptide as part of a single polypeptide chain by a peptide linker, a short sequence of amino acids 5, 10, 15, or 20 residues in length.

In particular embodiments, the peptide linker is a non-immunogenic sequence, such as a sequence rich in serine and glycine residues. In more particular embodiments, the peptide linker has the sequence SEQ ID NO 003.

In particular embodiments of the pharmaceutical composition for use in treating cancer, it is in the format of a dimer. Said dimer comprises a first monomer and a second monomer, and each monomer independently of the other monomer comprises an HLA fusion protein, an HLA heavy chain extracellular domain polypeptide fused to an Ig Fc portion, the latter of which may associate via disulfide bonds. Each monomer according to the invention is additionally associated with a B2m polypeptide.

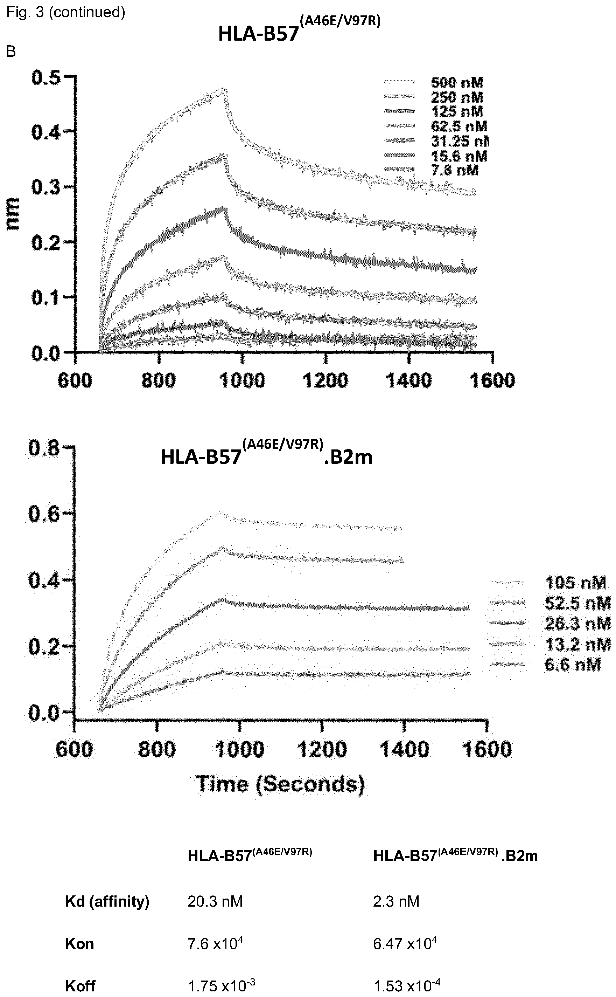

Natural HLA molecules expressed in the endoplasmic reticulum of human cells associate with peptide epitopes before undergoing transport and display on the cell surface. In particular embodiments of the pharmaceutical composition according to the invention, the HLA polypeptide is not associated with a peptide epitope. In other words, the antigen-binding groove, or antigen-binding cleft formed by the alpha 1 and alpha 2 domains of the HLA polypeptide is not bound to a small, antigenic peptide. Binding of peptide to HLA class molecules is thought to change their conformation, which may affect interactions with binding partners such as LILRB1 and LILRB2. The binding affinity and immunomodulatory effects of an HLA-B57 polypeptide associated with B2m polypeptide according to the invention, not further associated with a peptide epitope are demonstrated in Figures 3 to 11 of the Examples.

*Dosing and Administration of HLA fusion protein pharmaceutical compositions*

The pharmaceutical composition for use in treating malignant neoplastic disease according to the present invention comprises an HLA fusion protein associated with B2m polypeptide, and is typically formulated into pharmaceutical dosage forms to provide an easily controllable dosage of the drug and to give the patient an elegant and easily handled product. Said pharmaceutical composition further contains a pharmaceutically acceptable carrier. In further embodiments, the composition comprises at least two pharmaceutically acceptable carriers, such as those described herein.

The dosage regimen for the pharmaceutical composition of the present invention will vary depending upon known factors, such as the pharmacodynamic characteristics of the particular agent and its mode and route of administration; the species, age, sex, health, medical condition, and weight of the recipient; the nature and extent of the symptoms; the kind of concurrent treatment; the frequency of treatment; the route of administration, the renal and hepatic function of the patient, and the effect desired. In certain embodiments, the pharmaceutical composition of the invention may be administered in a single daily dose, or the total daily dosage may be administered in divided doses of two, three, or four times daily.

Many procedures and methods for preparing pharmaceutical compositions are known in the art, see for example L. Lachman et al. The Theory and Practice of Industrial Pharmacy, 4th Ed, 2013 (ISBN 8123922892).

*Combination medicaments*

Another aspect of the invention is pharmaceutical composition (comprising an HLA fusion protein: B2m polypeptide complex according to the first aspect of invention), formulated for administration in combination with a checkpoint inhibitory agent, wherein said checkpoint inhibitory agent is an antibody capable of binding to one of CTLA-4, PD-1, PD-L1, or PD-L2 with a dissociation constant of $10^{-7}$ mol/L or lower.

Another aspect of the invention is a checkpoint inhibitor agent for use in treating a malignant neoplastic disease, formulated for administration in combination with pharmaceutical composition comprising and HLA fusion protein according to the first aspect of the invention, wherein said checkpoint inhibitory agent is an antibody capable of binding to one of CTLA-4, PD-1, PD-L1, or PD-L2 with a dissociation constant of $10^{-7}$ mol/L or lower.

In particular embodiments of the pharmaceutical composition, or the checkpoint inhibitory agent according to the invention, the checkpoint inhibitory agent is capable of disrupting the inhibitory signaling cascade that limits immune cell activation, and in particular T cell activation.

Particularly, the checkpoint inhibitor agent is a non-agonist CTLA-4 ligand, a non-agonist PD-1 ligand, a non-agonist PD-L1 ligand, or a non-agonist PD-L2 ligand, which does not lead to attenuated T cell activity when binding to CTLA-4, PD-1, PD-L1 or PD-L2, respectively, on the surface on a T-cell. In certain embodiments, the term "non-agonist CTLA-4 ligand" or "non-agonist PD-1 ligand" covers both antagonists of CTLA-4 or PD-1 and ligands that are neutral vis-à-vis CTLA-4 or PD-1 signaling. In some embodiments, non-agonist CTLA-4 ligands used in the present invention are able, when bound to CTLA-4, to sterically block interaction of CTLA-4 with its binding partners CD80 and/or CD86 and non-agonist PD-1 ligands used in the present invention are able, when bound to PD-1, to sterically block interaction of PD-1 with its binding partners PD-L1 and/or PD-L2.

In particular embodiments, the checkpoint inhibitory agent disrupts inhibitory signaling cascades via a capacity to bind to CTLA-4 with a dissociation constant of at least, to mark the least affinity as expressed in $K_D$ value, $10^{-7}$ mol/L, or with stronger binding characterised by $K_D$ value of at least $10^{-8}$ mol/L, or even $10^{-9}$ mol/L, resulting in inhibition of the biological activity of its respective target. A non-agonist PD-1 ligand or a non-agonist PD-L1 (PD-L2) ligand in the sense of the invention refers to a molecule that is capable of binding to PD-1 (PD-L1, PD-L2) with a dissociation constant of at least $10^{-7}$ mol/L, particularly $10^{-8}$ mol/L or even $10^{-9}$ mol/L or lower, and which inhibits the biological activity of its respective target.

In further particular embodiments, the checkpoint inhibitory agent is a checkpoint inhibitor antibody selected from the clinically available antibody drugs ipilimumab (Bristol-Myers Squibb; CAS No. 477202-00-9), tremelimumab (CAS 745013-59-6), nivolumab (Bristol-Myers Squibb; CAS No 946414-94-4), pidilizumab (CAS No. 1036730-42-3), atezolizumab (Roche AG; CAS No. 1380723-44-3), avelumab (Merck KGaA; CAS No. 1537032-82-8), durvalumab (Astra Zeneca, CAS No. 1428935-60-7), and cemiplimab (Sanofi Aventis; CAS No. 1801342-60-8). In still more particular embodiments, the checkpoint inhibitor agent is pembrolizumab (Merck Inc.; CAS No. 1374853-91-4).

In certain embodiments, the combination therapy comprises two distinct dosage forms, for example, wherein said pharmaceutical composition comprising an HLA fusion protein is provided as a dosage form for intra-tumoral delivery, or local delivery in the vicinity of the tumor, for example, by subcutaneous injection, or intra-tumoral injection into a solid tumor, and said checkpoint inhibitor agent is provided as a dosage form for systemic delivery, particularly by intravenous injection. However, said checkpoint inhibitor agent and said pharmaceutical composition comprising an HLA fusion protein may also be delivered in two similar dosage forms.

Administration in combination, encompasses both simultaneous administration of the checkpoint inhibitor agent and the pharmaceutical composition comprising an HLA fusion protein, or in separate formulations, or administration of one substance immediately prior to, for example, in the week prior to, or at least in the month prior to, or immediately subsequent to, for example, in the week, or at most the month subsequent to, administration of a second substance.

## Medical treatment, Dosage Forms, Method of Manufacture

The pharmaceutical composition for use according to the invention is provided for use in treating various forms of cancer. Pre-clinical studies on other forms of LILBR2-directed antineoplastic therapy (one of several mechanism through which the pharmaceutical composition according to the invention is predicted to act) have demonstrated efficacy in renal cancer, and ovarian cancer. The safety and tolerability of LILRB2-targeting antibody MK-4830 is currently under investigation in a clinical trial targeting a wide range of solid organ cancers (mesothelioma, triple negative breast cancer, ovarian cancer, lung cancer, glioblastoma, pancreatic cancer, gastric cancer), in combination with chemotherapy (ClinicalTrials.gov Identifier: NCT03564691), all of which the inventors consider may feasibly be treated effectively by a pharmaceutical composition according to the invention.

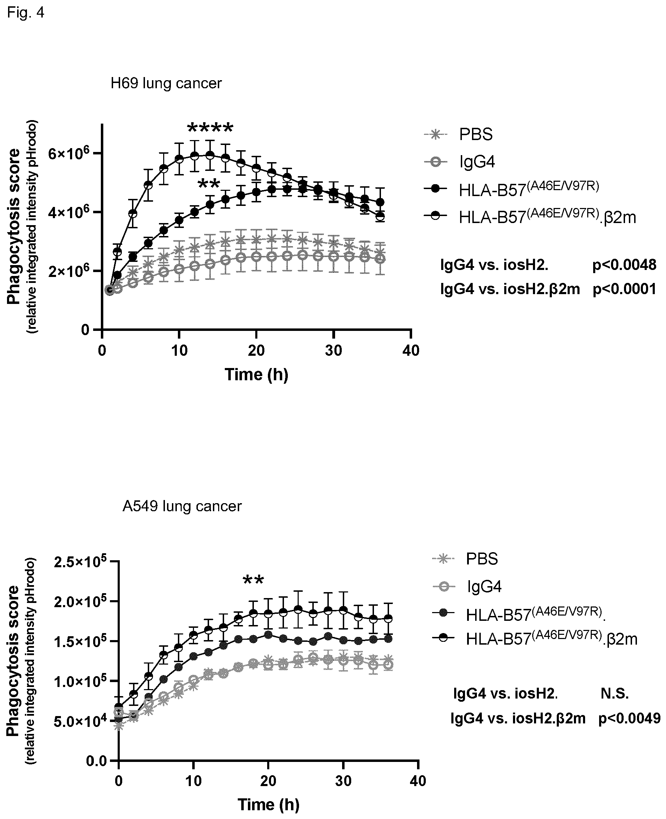

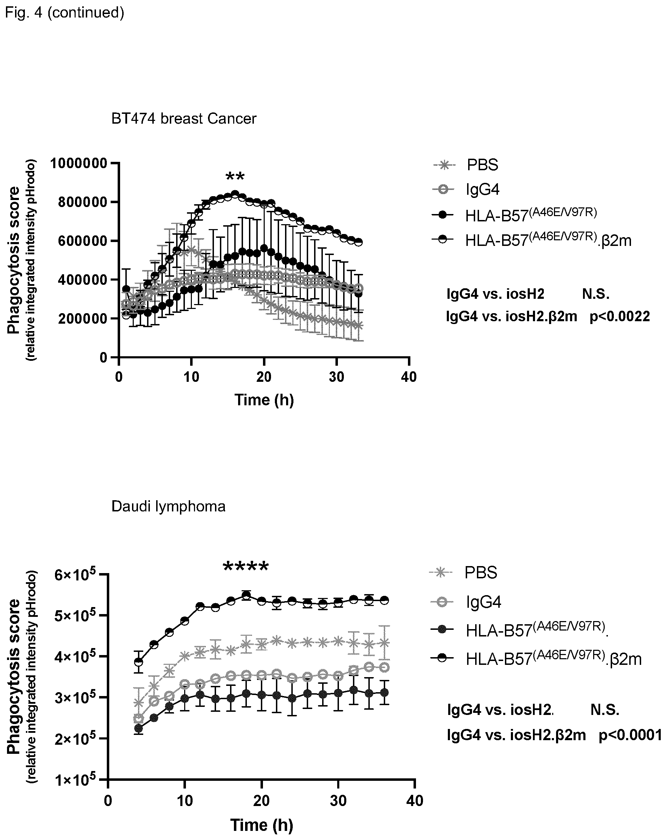

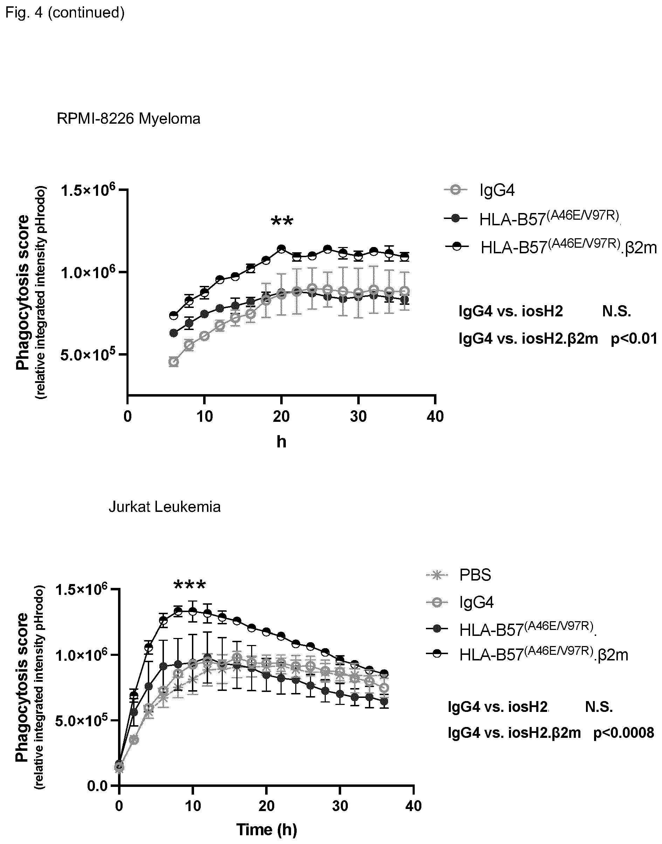

In particular embodiments of the pharmaceutical composition comprising an HLA fusion protein, it is provided for use to treat a type of liquid, or blood cancer. The inventors consider the characteristic T cell-exhaustion, and accessibility of circulating blood cancer cells to the T cell, and macrophage activation induced by pharmaceutical compositions according to the invention mean the treatment is likely to be effective. In particular such embodiments, the pharmaceutical composition is provided for use in a patient diagnosed with T cell leukemia. In other particular embodiments, the pharmaceutical composition is provided for use in a patient diagnosed with lymphoma, including, but not limited to, Burkitt's lymphoma as modelled by Daudi cells in the examples. In further embodiments, the pharmaceutical composition is provided for use in a patient diagnosed with multiple myeloma, as modelled by the RPMI-8226 cell line (Fig. 4).

In other particular embodiments, the pharmaceutical composition comprising an HLA fusion protein is provided for use in a patient diagnosed with a solid cancer, or a metastasis of a solid cancer. In some particular embodiments, the pharmaceutical composition is for use in a patient diagnosed with lung cancer. In particular embodiments, the lung cancer is a form of non-small cell lung cancer. In other particular embodiments, the lung cancer is a form of small cell lung cancer, or carcinoma. In other particular embodiments, the pharmaceutical composition comprising an HLA fusion protein is for use in a patient diagnosed with a form of breast cancer. In more particular embodiments, the cancer is estrogen receptor positive. In other particular embodiments, the cancer is progesterone receptor positive. In other particular embodiments, the cancer is human epidermal growth factor receptor 2 positive.

In some particular embodiments, the pharmaceutical composition comprising an HLA fusion protein, or the immune checkpoint inhibitor, is for use in a patient diagnosed with colon cancer. In particular embodiments, metastatic colon cancer.

In some embodiments of aspects of the invention relating to administration of a pharmaceutical composition according to the invention in combination with a checkpoint inhibition agent, it is provided for use in a form of malignant disease, or cancer, in which checkpoint inhibition therapy is approved for monotherapy, or combination therapy. In particular embodiments, the combination treatment is for use in a patient with colon cancer, particularly metastatic colon cancer. In other particular embodiments, the cancer is melanoma. In further particular embodiments, the cancer is pancreatic cancer. In still further particular embodiments the cancer is breast cancer.

Dosage forms may be for parenteral administration such as subcutaneous, intravenous, intrahepatic or intramuscular injection forms. Optionally, a pharmaceutically acceptable carrier and/or excipient may be present.

Wherever alternatives for single separable features are laid out herein as "embodiments", it is to be understood that such alternatives may be combined freely to form discrete embodiments of the invention disclosed herein.

*Description of the Figures*

Fig. 1  shows superior expression properties of HLA-B57$^{(A46E/V97R)}$ IgG4 fusion protein. RNA profiles of the indicated (A) HLA-B57 IgG4 fusion proteins and (B) β2m expressed from vectors within CHO cell clones. Fusion protein expression from clones transfected with HLA B57.β2m (DGC8-T39, DGC8-T64, & DGC8-73) and HLA-B57$^{(A46E/V97R)}$.β2m (DGC8-T54, DGC8-T75 & DGC8-91) on the basis of cell viability (C) and expressed protein titers (D). Table summarizes yield at the different transfection ratios tested.

Fig. 2  shows size exclusion chromatography (SEC) profiles of (A) HLA-B57 and (B) HLA-B57$^{(A46E/V97R)}$ constructs purified in three steps. From CHO supernatant A affinity purification B two methods diverge. Purification is performed with an additional step for B2m removal, followed by SEC C, and purification of B2m associated HLA-B57 is performed by direct SEC D. Inset summarized table (bottom) demonstrates yield of compounds purified from both constructs. HLA-B57.β2m has high amount of high molecular weight (HMW) species and also low molecular weight species (LMW), with reduced monomer content whereas HLA-B57$^{(A46E/V97R)}$.β2m has significantly reduced HMW, LWM and high monomer content. (C) shows recombinant protein yields from IgG Fc fusions of the indicated HLA-A30, B57, B58 and C08 wildtype and variant structures with the indicated amino acid substitutions at positions 46 and 97. CHO cells were transiently transfected with 2 vector constructs of: a) Fc fusion HLA molecules and b) human β2m, at a ratio 1:1. Expressed protein titers were obtained were quantified using Octet Red96 system (Sartorius) using protein A biosensors.

Fig. 3  (A) shows quantitative estimation of the binding affinities of LILRB2 with non-β2m-associated HLA-B57, non-β2m-associated HLA-B57$^{(A46E/V97R)}$ & HLA-B57$^{(A46E/V97R)}$.β2m measured by ELISA. non-β2m-associated HLA-B57 has an EC50 of 21 nM, non-β2m-associated HLA-B57$^{(A46E/V97R)}$ EC50 of 8.3 nM, and HLA-B57$^{(A46E/V97R)}$.β2m EC50 is 5.72 nM, demonstrating that amino acid substitutions do not reduce the binding of HLA-B57 heavy chain to LILRB2. (B) Quantitative estimation of the binding affinities of LILRB2 with non-β2m-associated HLA-B57$^{(A46E/V97R)}$ (Kd=20.3 nM) and HLA-B57$^{(A46E/V97R)}$.β2m (Kd=2.3 nM) measured by Bio-layer interferometry (BLI), confirming improved binding of the B57$^{(A46E/V97R)}$.β2m associated with β2m.

Fig. 4  shows HLA-B57$^{(A46E, V97R)}$ with or without β2m induced phagocytosis of liquid and solid cancer cells by human primary macrophages. Cancer cell lines derived from the indicated liquid and solid tumors were co-cultured with human primary macrophages, and phagocytosis of tumor cells was measured for 36 hours using IncuCyte live-cell imaging system. Experiments were repeated using at least 2 biologically independent samples. Error bars, SEM of n = 2 biological replicates with each containing 2 technical replicates. Statistical analysis was performed using 2-way ANOVA multiple comparison, followed by Dunnett's post-hoc analysis **p< 0.01, ***p< 0.001, ****p< 0.0001.

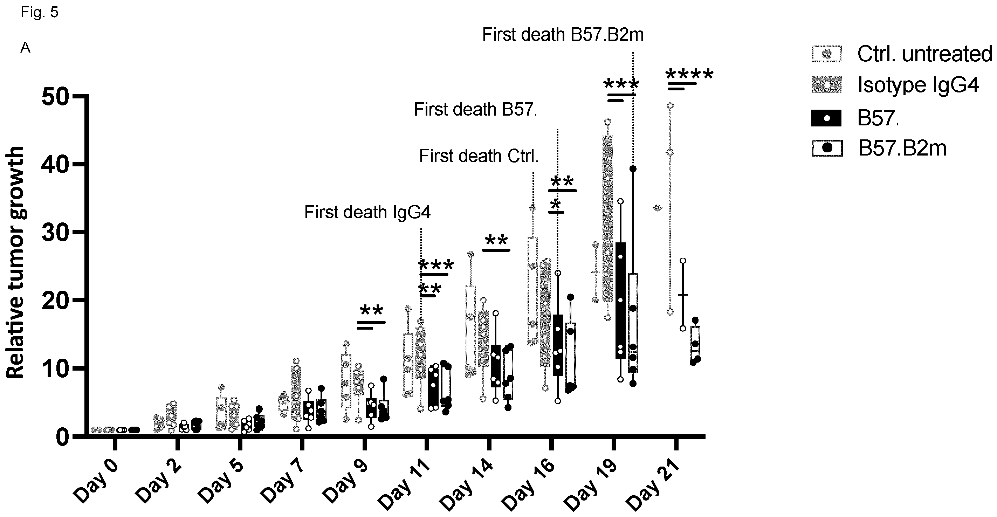

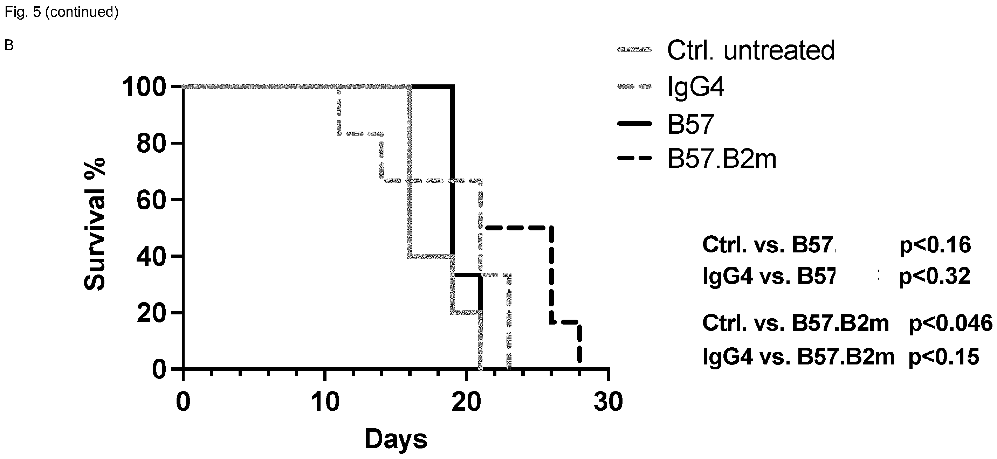

Fig. 5  shows that monotherapy with HLA-B57$^{(A46E, V97R)}$ with and withoutB2m (labelled B57 and B57.B2m respectively) reduces tumor growth, and HLA-B57$^{(A46E, V97R)}$.B2m increase survival in BRGSF-HIS humanized PDX lung cancer mice. A. Relative tumor growth (doubling time of tumor) and (B) survival following monotherapy HLA-B57$^{(A46E, V97R)}$ with and without β2m . I.p injections were performed every 5 days until end of study; concentration of injected compounds: isotype IgG4 (10 mg/kg), non-β2m-associated HLA-B57$^{(A46E, V97R)}$ (10 mg/kg), and HLA-B57$^{(A46E, V97R)}$.β2m (10mg/kg); n = 6. Data in A is plotted as box and whiskers showing all points min. to max. Statistical analysis for A, was performed using multiple comparison 2-way ANOVA (mixed effect model), followed by Tukey's post-hoc analysis where *p< 0.05, **p< 0.01, ***p< 0.001, ****p< 0.0001; Statistical analysis for survival B was performed using a Log-rank (Mantel-Cox) test.

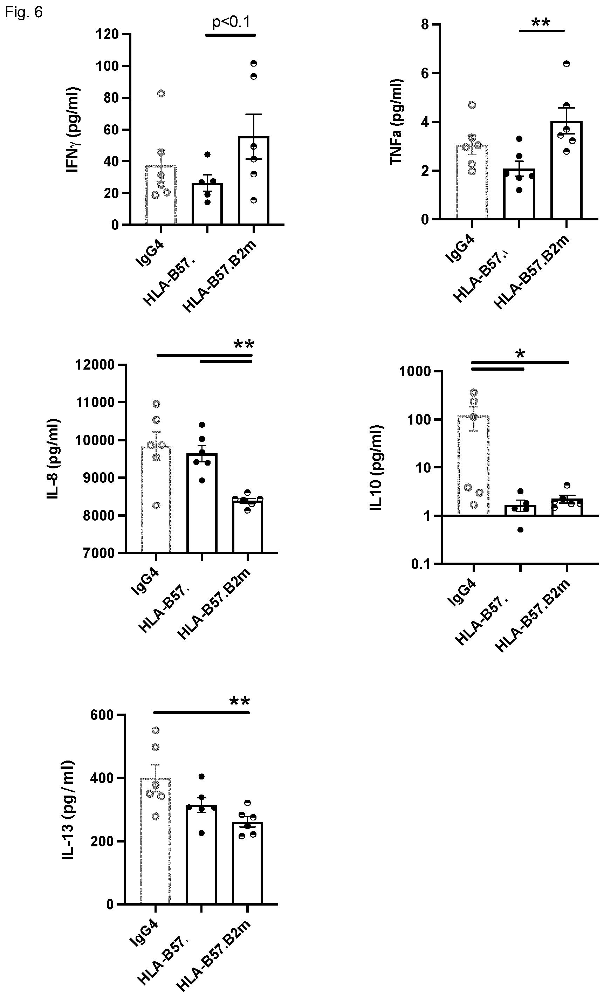

Fig. 6  shows the analysis of indicated cytokines in the blood of treated BRGSF-HIS humanized PDX lung cancer mice from Fig. 5 measured by V-Plex multiplexed cytokine immunoassay. Statistical analysis was performed using ordinary one-way ANOVA followed by Fischer's post-hoc analysis *p< 0.05, **p< 0.01, ***p< 0.001, ****p< 0.0001

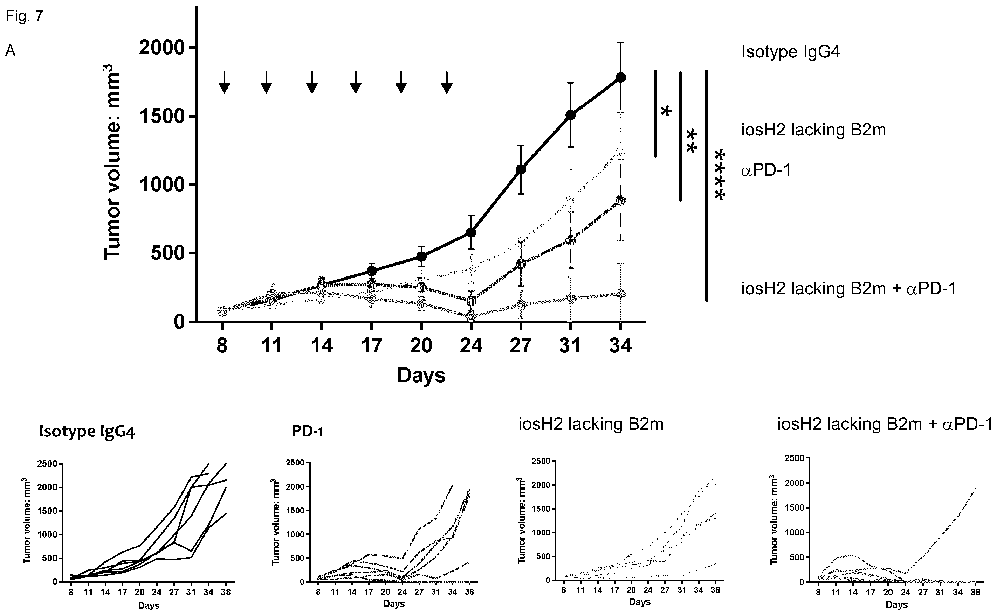

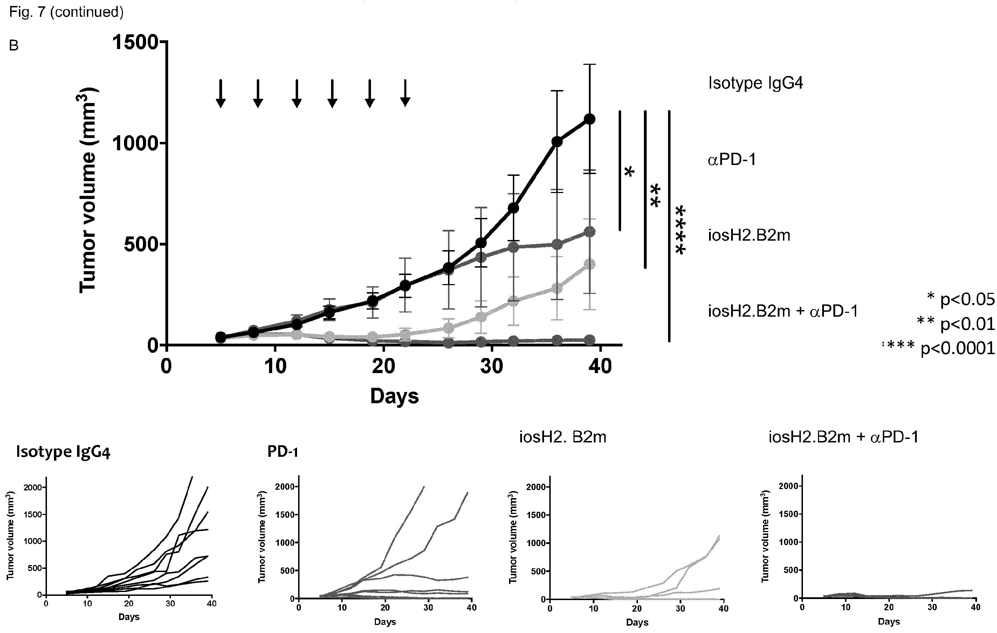

Fig. 7  shows that monotherapy of and combination therapy with anti-PD-1 checkpoint inhibitors and (A) non-β2m-associated (HLA-B57$^{(A46E/V97R)}$)and (B) β2m-associated HLA (HLA-B57$^{(A46E/V97R)}$.β2m) reduces the size of tumors in the C38 murine syngeneic colon carcinoma model. Mean (upper) tumor volume mm$^3$ of indi-

cated treated groups and (lower) spider plot showing individual animals and response to therapy for each group. Tumor volumes are expressed as mean $\pm$ SEM and analyzed by two-way ANOVA followed by Bonferroni post-hoc analysis, *p<0.05, **p<0.01, ****p<0.0001.

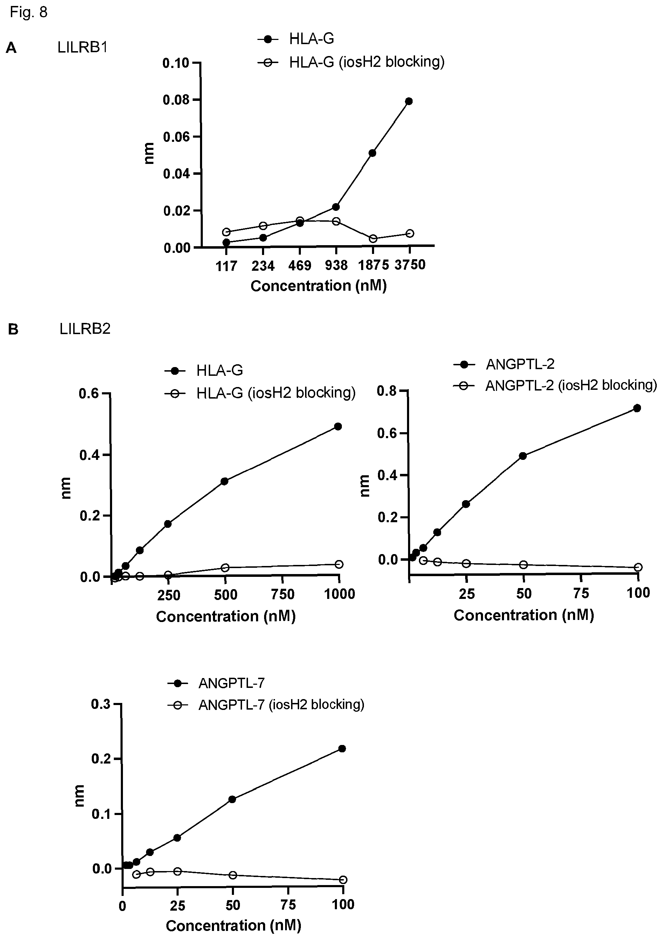

Fig. 8     shows HLA-B57$^{(A46E, V97R)}$.β2m (labelled iosH2) binds to LILRB1 and LILRB2, and blocks interactions to natural ligands. (A) iosH2 binds to human LILRB1 and competitively blocks the interaction to HLA-G. (B) iosH2 binds to human LILRB2 and competitively blocks the interaction to HLA-G (produced in house), ANGPTL2 (R&D systems, 9795-AN), and ANGPTL7 (R&D systems, 914-AN).

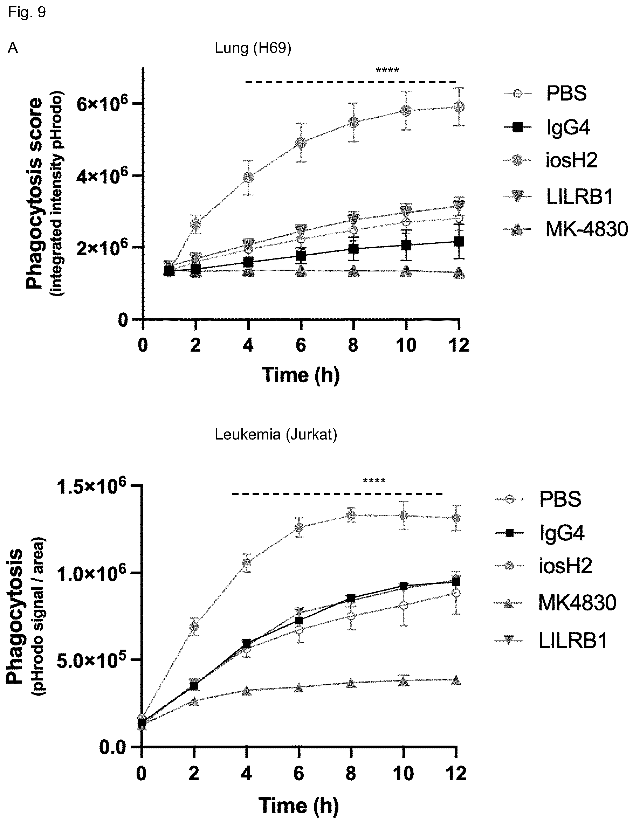

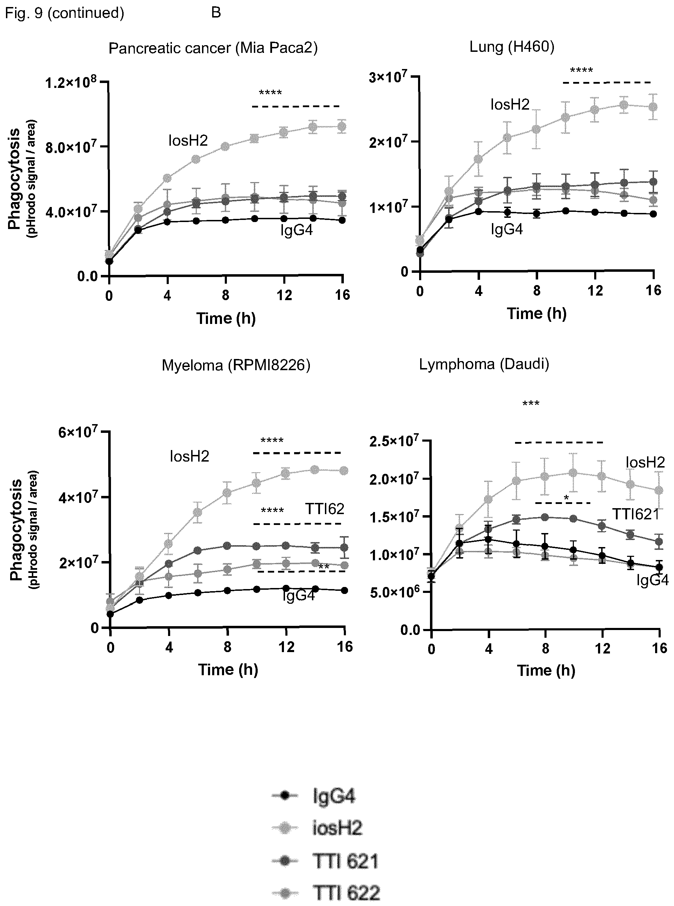

Fig. 9     shows HLA-B57$^{(A46E, V97R)}$.β2m (labelled iosH2) enhances primary macrophage mediated phagocytosis of cell lines derived from solid and liquid tumors. Cancer cell lines derived from various indications (Lung-H69, Leukemia-Jurkat, Pancreatic-MIA PaCa2, Lung-H460, Myeloma-RPMI8226, Lymphoma-Daudi) were co-cultured with human primary macrophages and phagocytosis was measured by IncuCyte live-cell imaging system. Phagocytosis was compared to (A) LILRB1 (Biolegend; Cat.#333722), LILRB2 (MK4830, US2018/0298096A1, ClinicalTrials.gov identifier NCT03564691), monoclonal antibodies and (B) against Trillium SIRPa Fc fusion proteins (TTI 621 (Lin G. PLos One 2017 12(10):e0187626) and TTI 622 (Clinical-Trials.gov identifier NCT03530683)).

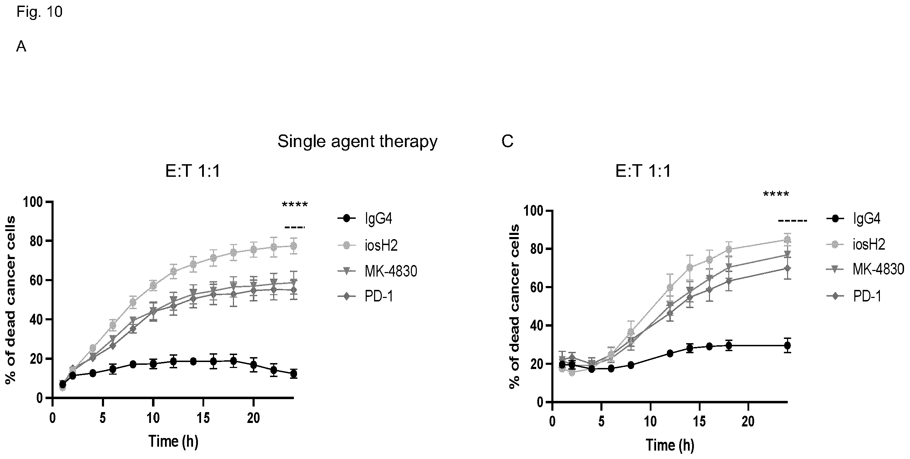

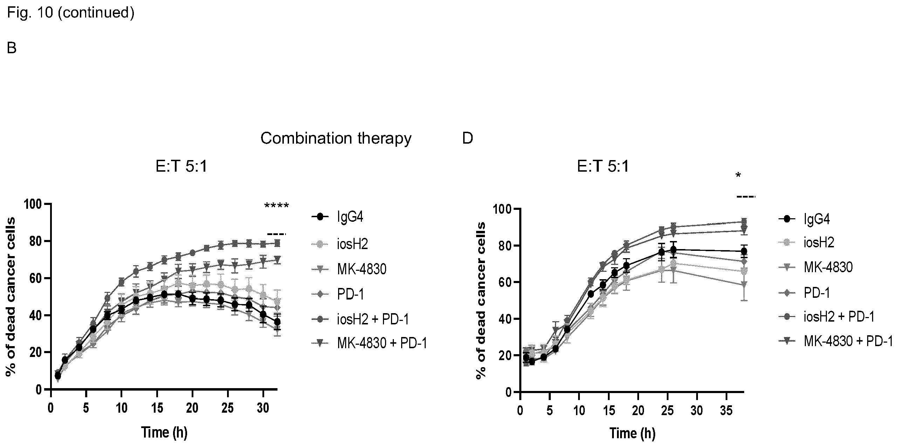

Fig. 10    shows HLA-B57$^{(A46E, V97R)}$.β2m (labelled iosH2) increases the killing activity of primary human T cells, as monotherapy and combination therapy with PD-1. Human primary T cells were incubated with (A,B) AML (THP1) and (C,D) Colon (HCT116) cancer cells, in a cell-contact manner at 2 different E:T (Effector cells: Target cells) ratios of 1:1 and 5:1, treated with compounds and the percentage of cancer cell killing was measured by IncuCyte live system.

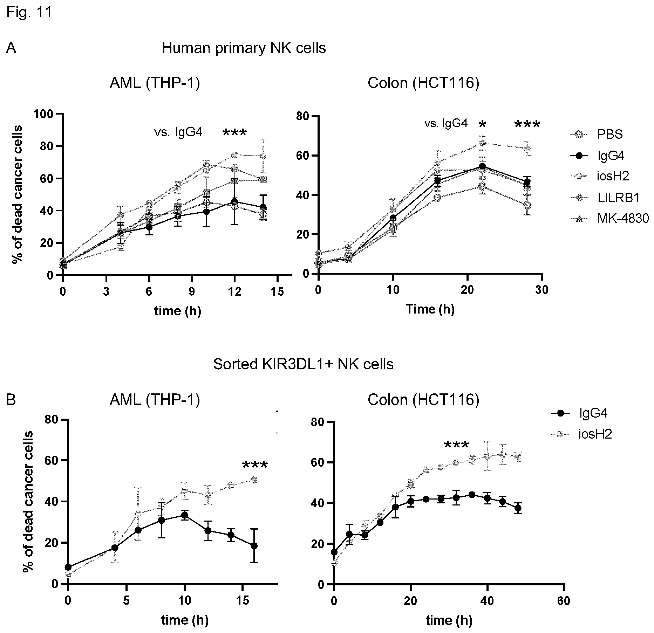

Fig. 11    shows HLA-B57$^{(A46E, V97R)}$. β2m (labeled iosH2) increases the killing potential of NK cells. (A) Isolated human primary NK cells were incubated with different cancer cell lines, treated with compounds and the percentage of cancer cell killing was measured by IncuCyte live system. (B) Human primary NK cells were sorted for KIR3DL1-positive population and same experiments as in (A) were repeated.

Examples

*Example 1. Generation of clone pools*

A first HLA fusion protein for medical use in humans was developed by the inventors by linking the heavy chain extracellular domain of the HLA-B57:01:01 polypeptide to an IgG4 Fc polypeptide (SEQ ID NO 002) to provide an HLA-fusion protein of SEQ ID NO 008. To increase the yield of this HLA fusion protein, inhibitory amino acids identified in the natural HLA-B57 extracellular domain amino acid sequence were altered by substitution of an alanine (A) residue at position 46 to glutamine (E), and a valine (V) at position 97 to an arginine (R), providing a variant HLA-B57 polypeptide (SEQ ID NO 001). This was fused to the IgG4 polypeptide (SEQ ID NO 002) via a linking peptide (SEQ ID NO 003), to provide a variant HLA-B57 fusion protein (SEQ ID NO 005). cDNA encoding the recombinant HLA-B57$^{(A46E/V97R)}$ fusion protein and the natural HLA-B57-derived fusion protein control, lacking the two mutations, were cloned into commercial expression vectors (Probiogen) downstream of a nucleic acid sequence encoding a secretion signal (SEQ ID NO 004). The vector constructs expressing HLA-B57-Fc & HLA-B57$^{(A46E/V97R)}$- Fc were co-transfected into Chinese hamster ovary (CHO) cells along with a plasmid comprising a nucleic acid encoding the β2m protein (SEQ NO 006) by microporation (MP) using the NEON Transfection Kit (Life Technologies #MPK10096). CHO-DG44 starter cells were transfected at different ratios of HLA fusion protein to β2m plasmid (4:1, 2:1, 1:1, 1:2). Selected clone pools were grown in standardized shaker flasks and with a defined cell seeding density of 4E5 vc/mL in 125 mL of PBG-CD-C4 supplemented medium including puromycin and methotrexate. Following adjusted selection pression with antibiotics, individual clone pools were selected for analysis. Measurement of viabilities and viable cell densities were performed using the Vi-CELL XR System, and Trypan blue cell exclusion method. Titer quantifications were measured at different time points (days) using an Octet RED machine (ForteBio, a Pall Division) with Protein A biosensors.

*Example 2. Purification of HLA-B57$^{(A46E/V97R)}$β2m and β2m removal procedure*

Equimolar RNA levels of the natural HLA-B57 or altered HLA-B57$^{(A46E/V97R)}$ fusion proteins relative to β2m were confirmed in selected clones cell clones (Fig. 1A). Analysis of clones expressing the HLA-B57 or variant fusion protein demonstrated that HLA-B57.β2m cell viability and titers are significantly lower than HLA-B57$^{(A46E/V97R)}$.β2m (Fig 1B and C). HPLC analysis of protein collected from supernatants of each demonstrate that HLA-B57.β2m has a high amount of high molecular weight (HMW) species and also low molecular weight species (LMW), with reduced monomer content, whereas HLA-B57$^{(A46E/V97R)}$.β2m has significantly reduced HMW, LWM and high monomer content (Fig 2A and B), demonstrating the increased stability of the variant HLA heavy chain.

The HLA-B57$^{(A46E/V97R)}$.β2m complex was then isolated from filtered CHO cell supernatants by affinity column purification. Purification of proteins and removal of β2m under acidic conditions was performed as a two-step purification protocol. As a first step, Protein G Sepharose [(4 Fast Flow) Sigma, #GE-17-0618-01)] beads were used to capture HLA-B57$^{(A46E/V97R)}$ associated with β2m from supernatants. After an overnight incubation at 4 degrees on a rocker, the recovered beads were washed in PBS, and subsequently HLA-B57$^{(A46E/V97R)}$ fusion proteins were eluted using standard IgG-Elution Buffer (pH 2.8) (Pierce™ IgG Elution Buffer, Thermo Fischer #21004). A second step of size exclusion chromatography-based purification was performed to separate HLA-B57$^{(A46E/V97R)}$ from β2m under acidic conditions, to provide an non-β2m-associated HLA fusion protein. A Superdex 10/300 gel filtration column, pre-equilibrated in Sodium Citrate (100 mM, pH 3.0) was used for the separation. An injection of 0.5 ml of the protein at 2.0 mg/ml concentration was applied, and the desired non-β2m-associated HLA-B57$^{(A46E/V97R)}$. protein peak eluted at 12.7 ml and the peak for β2m eluted at 22.0 ml. Fig. 2 shows the yields of various stages of the process described above, indicating that the separation of β2m to yield a non-β2m-associated HLA fusion protein results in a loss of approximately 50% of the immunomodulatory HLA-B57$^{(A46E/V97R)}$ fusion protein.

To confirm the importance of 46E and 97R residues for optimal recombinant expression of various HLA class I heavy chains associated with differing immune phenotypes in the human population, the inventors measured the impact of these amino acid substitutions on additional IgG Fc fusion protein constructs comprising other representative HLA class I heavy chain polypeptide sequences associated with immunogenic effects in the human population, HLA-A30, HLA-B58, and HLA-C08 (Fig. 2C). Using the same process as for HLA-B57 IgG4 fusion proteins, nucleic acid expression vectors encoding alternative immunogenic HLA class I heavy chains IgG4 fusion proteins were created: HLA-A30, A*30:01:01:01 (SEQ ID NO 007), HLA-B57 B*57:01:01:01 (SEQ ID NO 008), HLA-B58, B*58:01:01:01 (SEQ ID NO 009), and HLA-C08, C*08:02:01:01 (SEQ ID NO 010). Next, modified constructs were created introducing to measure the impact amino acid substitutions adding, or removing an E46 amino acid residue, or an R97 residue into the HLA heavy chain extracellular domain portion of each HLA-Fc fusion protein as follows: HLA-A30$^{E46A}$ (SEQ ID NO 011), HLA-A30$^{I97R}$ (SEQ ID NO 012), HLA-A30$^{E46A/I97R}$ (SEQ ID NO 013), HLA-B57$^{A46E}$ (SEQ ID NO 014), HLA-B57$^{V97R}$ (SEQ ID NO 015), HLA-B57$^{A46E/V97R}$ (SEQ ID NO 005), HLA-B58$^{E46A}$ (SEQ ID NO 016), HLA-B58$^{R97V}$ (SEQ ID NO 017), HLA-B58$^{E46A/R97V}$ (SEQ ID NO 018), HLA-C08$^{E46A}$ (SEQ ID NO 019), HLA-C08$^{R97V}$ (SEQ ID NO 020), HLA-C08$^{E46A/R97V}$ (SEQ ID NO 021). The production yield of recombinantly expressed β2m-associated HLA constructs by CHO cells transiently transfected with nucleic acid expression vectors encoding wildtype and variant HLA heavy chains IgG4 fusion proteins and β2m, was assessed in the supernatant of CHO cells using Protein A biosensors (Octet Red96 system, Sartorius).

These results confirmed that in all molecules tested, HLA heavy chains characterized by both amino acid 46E and 97R residues were associated with optimal recombinant protein yields, and that introducing both an A46E and a V97R substitution into the HLA-B57 heavy chain sequence achieved the highest yield among all constructs. Conversely, the introduction of both 46A and 97V present in wildtype HLA-B57 into HLA-A30, HLA-B58, or HLA-C08 significantly reduced productivity yields, confirming that amino acids 46E and 97R are key for stabilization and production of optimal titers of HLA heavy chains, including a variety HLA-Fc molecules associated with β2m.

*Example 3. Quantification of the interaction of LILRB2 with HLA-857$^{(A46E/V97R)}$ and HLA-B57$^{(A46E/V97R)}$ with or without β2m.*

Considering the large loss of yield that accompanies the purification of HLA relative to its parent HLA still associated with β2m polypeptide, the inventors went on to dissect the immunological properties most relevant to tumor immunity associated with each HLA-B57$^{(A46E/V97R)}$ format. As a first step, the impact the removal of β2m on from an HLA-B57 IgG4 fusion protein on binding to the innate immune receptor LILRB2 was examined.

The quantification of the affinity of interaction of LILRB2 with non-β2m-associated HLA-B57 and HLA-B57$^{(A46E/V97R)}$, and HLA-B57$^{(A46E/V97R)}$.β2m was measured using the enzyme-linked immunosorbent assay (ELISA) method. Flat bottom Pierce™ Streptavidin coated high binding capacity 96 well plates (Pierce #15500) were coated with 50 μl of c-terminally biotinylated antigen molecules (LILRB2, BPS Bioscience #100335) immobilized at a final concentration of 5 μg/ml in PBS buffer. PBS and IgG isotype were used as negative controls. A serial dilution of non-β2m-associated HLA-B57 and HLA-B57$^{(A46E/V97R)}$, and HLA-B57$^{(A46E/V97R)}$.β2m (eight concentration points: 10, 2.5, 1, 0.25, 0.1, 0.025, 0.01, 0.0025 μg/ml) was applied (50 μl) in duplicates. An APC conjugated goat anti-human IgG antibody (Jackson Immuno Research #109-135-098) with 1:100 dilution in TBS (50 μl) was used for detection. Finally, 50 μl TBS in each well was added and a fluorescence scan was performed with APC excitation and emission wavelengths of 650 nm & 660 nm, respectively. Graphpad Prism v9.1.2 and the three-parameter based log (agonist) vs. response model was used to determine the EC50 of the interaction with LILRB2 (Fig. 3A).

The binding of non-β2m-associated HLA-B57$^{(A46E/V97R)}$, and HLA-B57$^{(A46E/V97R)}$.β2 was also assessed by Bio-layer interferometry (BLI). Octet Red 96e (Sartorius) based Bio-Layer Interferometry Technology (BLI) was used for the quantification of binding affinities of HLA fusion proteins with LILRB2 and for blocking experiments using HLA-G and ANGPTL2/7. The biotinylated LILRB2 (BPS Bioscience) protein was immobilized on streptavidin (SAXS) biosensors. The biosensors were incubated with increasing concentrations (500, 250, 125, 62.5, 31.25, 15.6 and 7.8nM) of the non-$\beta$2m-associated HLA-B57$^{(A46E/V97R)}$ and (105, 52.5, 26.3, 13.2, and 6.6nM) of HLA-B57$^{(A46E/V97R)}$:$\beta$2m and interaction and reference sensograms were recorded (Fig. 3B). For blocking experiments biotinylated LILRB2 sensors were incubated with 1 or 4 $\mu$M concentrations of HLA-B57$^{(A46E/V97R)}$:$\beta$2m, followed by increasing concentrations of HLA-G (3750 to 12.5 nM), or ANGTPL2 and ANGTPL7 (100 to 1.56 nM) (Fig. 8). The data analysis, double reference subtraction and quantification of the binding affinities and kinetic parameters were determined using Data Analysis HT 12.0.2.59 software package and data were fitted locally using a bivalent analyte model (2:1 model) for ligand-analyte reaction.

Together, the binding assays demonstrated improved binding of the variant HLA fusion proteins to LILRB2, as HLA lacking association with $\beta$2m, and particularly when associated with $\beta$2m, suggesting the variant HLA heavy chain HLA-B57$^{(A46E/V97R)}$ has high immunomodulatory potential (Fig. 3 and Fig. 8).

*Example 4. Increased killing of tumor cells in vitro*

Next, the capacity of the $\beta$2m polypeptide to influence HLA-B57$^{(A46E, V97R)}$ IgG4 fusion protein induction of phagocytosis of tumor cells by human primary macrophages was assessed towards liquid (lymphoma, leukemia and myeloma) and solid (breast and lung cancer) cancer cells. Cancer cell lines derived from the indicated liquid and solid tumors were co-cultured with human primary macrophages, and phagocytosis of tumor cells was monitored according to the manufacturer's instructions for 36 hours using IncuCyte live-cell imaging system. Primary human donor-derived monocytes were isolated from PBMCs from healthy donors and differentiated into macrophages by 5-7 days of culture in specific macrophage culture medium. On day 1 post plating compounds were added to wells at 10ug/ml (isotype controls) or 20ug/ml (HLA fusion protein). On day 5-7 post plating, compounds were added once again to the macrophages and two downstream experiments were performed to determine phagocytosis potential of macrophages using live-cell imaging.

Cancer cells were stained with CellTrace™ CFSE (ThermoFisher) according to manufacturer's instructions and subsequently 1000 cells/well were plated in flat-bottom 96 well plates (Greiner) together with 1000-5000 primary T cells. Media contained 250nM of Cytotox Red (Sartorius). Live cell imaging was performed using the Incucyte S3 Live-Cell Analysis System (Sartorius). 4 non-adjacent images per well were analysed with Incucyte software v2020C. The CFSE signal was segmented in green objects, and every object was counted as cancer cell. Dead cells were identified with Cytotox signal segmented in red objects. Cancer cell death was detected by colocalization of green and red objects. Results demonstrate that HLA-B57$^{(A46E, V97R)}$.B2m increases the activity of macrophage phagocytosis against cancer cells, while HLA-B57$^{(A46E, V97R)}$ lacking $\beta$2m shows slightly reduced activity (Fig 4).

*Example 5 immunomodulation of tumor growth in vivo*

Non-$\beta$2m-associated HLA-B57$^{(A46E, V97R)}$ and HLA-B57$^{(A46E, V97R)}$.B2m were then each assessed for impact on tumor growth *in vivo* in BRGSF-HIS mice (Rag2$^{-/-}$, IL-2R$\gamma^{-/-}$, Flk2$^{-/-}$, bearing a NOD specific SIRPa mutation inhibiting murine endogenous macrophages). These recipients were humanized with intrahepatic injection of purified human umbilical cord blood derived CD34+ cells, and implanted with patient-derived xenograft (PDX) lung cancer cells. These mice comprise all the major human immune cell subsets, including T and B cells, NK cells, and myeloid cells. The non-small cell lung cancer tumors used for xenograft were assessed for expression of the HLA fusion protein target LILRB2 after transplantation in the lung using a commercially available immunohistochemistry antibody, and positive expression following engraftment was confirmed, demonstrating this is an effective model for assessing human LILRB2-mediated immunomodulatory effects.

LU6425 Lung PDX NSCLC tumor fragments were obtained frozen from CrownBio's HuPrime® PDX collection. LU6425 tumor is derived from a western female, age 69. Pathology diagnosis: adenocarcinoma of the lung. Eighteen BRGSF-HIS humanized mice were obtained for efficacy studies, humanization was performed with 6 different HSCs CD34+ donors, with cut-off engraftment of >30% human CD45+ cells (Genoway). LU6425 PDX tumors were expanded in BRGSF mice, PDX fragments are stored frozen in RPMI1640:FBS:DMSO (6:3:1) in liquid nitrogen until use. The fragments were thawed at 37°C for 5 min, rinsed twice in culture media RPMI 1640 medium (ref. L0500-500, Dutscher or equivalent). Ten (10) female BRGSF mice were subcutaneously implanted into the left flank with LU6425 tumor fragments. When tumor volumes of mice from the in vivo amplification phase reached 500-1000 mm3 were surgically excised and tumor fragments (2-3mm3) were subcutaneously implanted into the left flank of eighteen (18) female BRGSF-HIS mice. The day of tumor implantation is considered as day 0 (D0). Once tumors were established, mice received 4 intraperitoneal injections of Flt3 ligand (10 $\mu$g per injection) over 7 days. In this protocol, the Flt3 ligand expands the myeloid cell population. Flt3 injections were started 1 day before treatment onset, when tumors reached a mean volume of ~30- 250mm3. 6 mice each were allocated to IgG4 isotype control, non-$\beta$2m-associated HLA-B57$^{(A46E, V97R)}$. or HLA-B57$^{(A46E, V97R)}$.$\beta$2m treatment groups with uniform mean tumor volume between groups. The treatment was administered by injection into the peritoneal cavity (IP). The administration volume was 10 mL/kg adjusted to the most recent individual body weight. Mice were euthanized at a cutoff tumor volume. Animal welfare for this study complies with the UK Animals Scientific Procedures Act 1986 (ASPA) in line with Directive 2010/63/EU of the European Parliament and the Council of 22 September 2010 on the protection of animals used for scientific purposes. All experimental data management and reporting procedures were in strict accordance with applicable Crown Bioscience UK Guidelines and Standard Operating Procedures.

Both HLA fusion protein compounds were injected intraperitoneally every five days for the duration of the experiment. The relative tumor growth (doubling time) was significantly reduced by both non-β2m-associated HLA-B57$^{(A46E, V97R)}$ and HLA-B57$^{(A46E, V97R)}$. β2m monotherapy (Fig. 5A). However, HLA-B57$^{(A46E, V97R)}$. β2m monotherapy also significantly extended the survival of treated mice vs. controls in this highly relevant cancer model for human immunotherapy (Fig. 5B). The concentration of cytokines in the plasma of treated BRGSF-HIS humanized PDX lung cancer mice collected at terminal stage, and was then assessed using a V-PLEX Proinflammatory Panel 1 Human MSD (MSD). HLA-B57$^{(A46E, V97R)}$. β2m significantly modulated several blood cytokines following therapy in mice, while non-β2m-associated HLA-B57$^{(A46E, V97R)}$ monotherapy altered only IL-10 levels compared to the control IgG4 treated group, confirming that an HLA fusion protein associated with β2m was a stronger immunomodulator in this cancer model (Fig. 6).

*Example 6 Combination with checkpoint inhibitors*

Both HLA fusion protein compounds with or without associated β2m were next tested for their ability to complement checkpoint inhibitor therapy with a PD-1 neutralizing antibody. C38 tumor fragments were injected subcutaneously into the right flanks of syngeneic female C57BL/6 mice. Once the tumor reached ±50 mm$^3$, animals were equally distributed according among groups with equivalent mean tumor volume size. Tumor diameters were measured using a caliper over the course of the study, and volume was calculated according to the formula, $D/2 \times d^2$ where D and d correspond to the longest and shortest diameter of the tumor in mm, respectively. The experimental design of injection time points of cells and injection of substances was as follows: isotype IgG4 (10mg/Kg) bi-weekly x 3; non-β2m-associated HLA-B57$^{(A46E/V97R)}$ (5 mg/Kg) bi-weekly x 3; HLA-B57$^{(A46E/V97R)}$.β2m (5 mg/kg) bi-weekly x 2. Some groups received simultaneous combination treatment with 10mg/kg injections of anti-PD-1 (RMP1-14) In this murine model, both compounds provided some advantage to counter tumor growth, however pharmaceutical compositions comprising β2m-associated HLA fusion protein more effectively controlled tumor growth in combination with an antiPD-1 antibody in all animals in the group, whereas a single instance of tumor escape and growth was observed in the equivalent non-β2m-associated HLA fusion protein group (Fig. 7).

*Example 7 Mechanisms of anti-tumour activity of HLA-B57$^{(A46E/V97R)}$.β2m*

HLA-B57 has the capacity to bind to multiple inhibitory immune molecules, including LILRB1, LILRB2 and KIR3DL1. LILRB1/2 and KIR3DL1 are expressed in diverse sets of immune cells, this including LILRB1 expression in myeloid cells (e.g. macrophages), T cells, NK cells, B cells, and tumors, LILRB2 expression in myeloid cells (e.g. macrophages), T cells and tumors, and KIR3DL1 expression in NK cells, where binding with cell-bound ligands such as HLA-G or MHC family molecules expressed by tumor cells, or soluble inhibitory factors such as angiopoietin-like proteins (ANGPTL) is thought to inhibit cytolytic function. Ligation of LILRB1/2 expressed by macrophages is thought to drive differentiation of suppressive myeloid cell subsets such as M2 macrophages and myeloid-derived suppressor cells (MDSC), both of which are associated with permissive growth of tumor cells.

The inventors investigated whether HLA-B57$^{(A46E/V97R)}$.β2m acts by multimodal inhibition of LILRB1, LILRB2 and KIR3DL1 receptors. HLA-B57$^{(A46E/V97R)}$.β2m (iosH2) binding to LILRB1, LILRB2 and KIR3DL1 was confirmed by biolayer interferometry. Reduced SHP1, and SHP2 phosphorylation upon exposure of primary human macrophages to HLA-B57$^{(A46E/V97R)}$.β2m suggested that it acts as an antagonist of negative regulation of the immune response via these molecules. The ability of HLA-B57$^{(A46E/V97R)}$.β2m to block binding of LILRB1/2 with various natural ligands was then assessed. Indeed HLA-B57$^{(A46E/V97R)}$.β2m (iosH2) competitively blocked the interaction of LILRB1 to HLA-G, as well as LILRB2 with HLA-G, ANGPTL2, and ANGPTL7 (Fig. 8).

Next, the investigators assessed whether HLA-B57$^{(A46E/V97R)}$.β2m could enhance the capacity of primary human macrophages to phagocytose tumor cells. HLA-B57$^{(A46E/V97R)}$.β2m induced superior phagocytosis of lung cancer, blood cancer (myeloma or leukemia), and pancreatic cancer compared to existing macrophage checkpoint targeting molecules, such as monoclonal antibodies targeting LILRB1 or 2 (Fig. 9A), or signal regulatory protein alpha (SIRPa) IgFc fusion protein constructs (Fig. 9B). In addition, HLA-B57$^{(A46E/V97R)}$.β2m increased the tumor cell-killing activity of primary human T cells, both as a single agent, or as a combination therapy with anti-PD-1 checkpoint inhibition (Figure 10), inducing improved tumor killing even at very low ratios of effector to target cells compared to monoclonal anti-LILRB2 antibody. Lastly, the ability of the construct to influence NK cells was assessed. HLA-B57$^{(A46E/V97R)}$.β2m was demonstrated to increase the killing potential of NK cells to a greater extent than monoclonal antibodies specific for LILRB1 or 2 (Fig. 11A). The effect was stronger in a culture of sorted KIR3DL1+ NK cells compared to total NK cells, suggesting HLA-B57$^{(A46E/V97R)}$.β2m binding to KIR3DL1 played a role in this enhanced tumor killing (Fig. 11B).

*Summary*

Unexpectedly, the data demonstrated that an HLA-B57$^{(A46/V97)}$ IgG4 fusion protein was effective at impeding tumor growth in vivo either with, or without the presence of β2m. To the inventor's knowledge, this is the first time that the utility of β2m association with human HLA fusion proteins has been examined in comprehensive models examining the human lymphoid and myeloid cells which are an important target for HLA fusion protein function both *in vitro* and *in vivo.* As HLA heavy chain fusion proteins have considerably higher yields due to the shorter production process (Fig. 2), these finding suggests it may be desirable to use pharmaceutical composition comprising an HLA molecule and a β2m molecule according to the invention in antineoplastic therapeutic settings including, but not limited to epithelial cancers such as lung cancer, colon cancer, and breast cancer, and blood cell malignancies such as myeloma, lymphoma, and leukemia. Mechanistic investigations suggest that the capacity of HLA-B57$^{(A46E/V97R)}$.β2m to antagonize multiple inhibitory pathways on macrophages, T cells, and NK cells underlies its profound antitumor activity compared to existing agents which target single immune-inhibitory pathways.

<u>Citations:</u>

Arosa et al. Trends in Immunology 2007 Mar; 28(3):115-23

WO 2017153438 A1; WO2016124661A1; WO2018 029284 A1.

<u>SEQUENCES</u>

SEQ ID NO 001        variant HLA-B57:01 extracellular domain A46E V97R (synthetic construct)

*GSH*SMRYFYTAMSRPGRGEPRFIAVGYVDDTQFVRFDSDAASPRM**E**PRAPWIEQEGPEYWDG

ETRNMKASAQTYRENLRIALRYYNQSEAGSHIIQ**RM**YGCDVGPDGRLLRGHDQSAYDGKDYIAL

NEDLSSWTAADTAAQITQRKWEAARVAEQLRAYLEGLCVEWLRRYLENGKETLQRADPPKTHV

THHPISDHEATLRCWALGFYPAEITLTWQRDGEDQTQDTELVETRPAGDRTFQKWAAVVVPSG

EEQRYTCHVQHEGLPKPLTLRWEPSSQS

SEQ ID NO 002        Optimized IgG4 Fc (synthetic construct)

ESKYGPPCPPCPAPEFLGGPSVFLFPPKPKDTLMISRTPEVTCVVVDVSQEDPEVQFNWYVDGV

EVHNAKTKPREEQFNSTYRVVSVLTVLHQDWLNGKEYKCKVSNKGLPSSIEKTISKAKGQPREP

QVYTLPPSQEEMTKNQVSLTCLVKGFYPSDIAVEWESNGQPENNYKTTPPVLDSDGSFFLYSRL

TVDKSRWQEGNVFSCSVMHEALHNHYTQKSLSLSLG

SEQ ID NO 003        peptide linker (synthetic construct)
GGGGSGGGGS
SEQ ID NO 004        secretory signal (synthetic construct)
AAAMNFGLRLIFLVLTLKGVQC
SEQ ID NO 005        variant HLA-B57:01 extracellular domain A46E V97R IgG4 fusion protein (synthetic construct)

GSHSMRYFYTAMSRPGRGEPRFIAVGYVDDTQFVRFDSDAASPRMEPRAPWIEQEGPEYWDG

ETRNMKASAQTYRENLRIALRYYNQSEAGSHIIQRMYGCDVGPDGRLLRGHDQSAYDGKDYIAL

NEDLSSWTAADTAAQITQRKWEAARVAEQLRAYLEGLCVEWLRRYLENGKETLQRADPPKTHV

THHPISDHEATLRCWALGFYPAEITLTWQRDGEDQTQDTELVETRPAGDRTFQKWAAVVVPSG

EEQRYTCHVQHEGLPKPLTLRWEPSSQS*GGGGSGGGGS*ESKYGPPCPPCPAPEFLGGPSVFL

*FPPKPKDTLMISRTPEVTCVVVDVSQEDPEVQFNWYVDGVEVHNAKTKPREEQFNSTYRVVSVL*

*TVLHQDWLNGKEYKCKVSNKGLPSSIEKTISKAKGQPREPQVYTLPPSQEEMTKNQVSLTCLVK*

*GFYPSDIAVEWESNGQPENNYKTTPPVLDSDGSFFLYSRLTVDKSRWQEGNVFSCSVMHEALH*

*NHYTQKSLSLSLG*

SEQ ID NO 006.        B2m (homo sapiens)

EP 4 380 968 B1

IQRTPKIQVYSRHPAENGKSNFLNCYVSGFHPSDIEVDLLKNGERIEKVEHSDLSFSKDWSFYLLYYTEFT

PTEKDEYACRVNHVTLSQPKIVKWDRDM

NEW SEQUENECES VARIANT HLA-FC

SEQ ID NO 007         HLA-A30 IgG4 Fc fusion protein (synthetic construct)

GSHSMRYFSTSVSRPGSGEPRFIAVGYVDDTQFVRFDSDAASQRMEPRAPWIEQERPEYWDQ

ETRNVKAQSQTDRVDLGTLRGYYNQSEAGSHTIQIMYGCDVGSDGRFLRGYEQHAYDGKDYIA

LNEDLRSWTAADMAAQITQRKWEAARWAEQLRAYLEGTCVEWLRRYLENGKETLQRTDPPKT

HMTHHPISDHEATLRCWALGFYPAEITLTWQRDGEDQTQDTELVETRPAGDGTFQKWAAVVVP

SGEEQRYTCHVQHEGLPKPLTLRWELSSQP<u>GGGGSGGGGS</u>*ESKYGPPCPPCPAPEFLGGPSV*

*FLFPPKPKDTLMISRTPEVTCVVVDVSQEDPEVQFNWYVDGVEVHNAKTKPREEQFNSTYRVVS*

*VLTVLHQDWLNGKEYKCKVSNKGLPSSIEKTISKAKGQPREPQVYTLPPSQEEMTKNQVSLTCL*

*VKGFYPSDIAVEWESNGQPENNYKTTPPVLDSDGSFFLYSRLTVDKSRWQEGNVFSCSVMHEA*

*LHNHYTQKSLSLSLG*

Extracellular domain of HLA-A*30:01, <u>peptide linker</u>, *IgG4 Fc*

SEQ ID NO 008         HLA-B57 IgG4 Fc fusion protein (synthetic construct)

GSHSMRYFYTAMSRPGRGEPRFIAVGYVDDTQFVRFDSDAASPRMAPRAPWIEQEGPEYWDG

ETRNMKASAQTYRENLRIALRYYNQSEAGSHIIQVMYGCDVGPDGRLLRGHDQSAYDGKDYIAL

NEDLSSWTAADTAAQITQRKWEAARVAEQLRAYLEGLCVEWLRRYLENGKETLQRADPPKTHV

THHPISDHEATLRCWALGFYPAEITLTWQRDGEDQTQDTELVETRPAGDRTFQKWAAVVVPSG

EEQRYTCHVQHEGLPKPLTLRWEPSSQS<u>GGGGSGGGGS</u>*ESKYGPPCPPCPAPEFLGGPSVFL*

*FPPKPKDTLMISRTPEVTCVVVDVSQEDPEVQFNWYVDGVEVHNAKTKPREEQFNSTYRVVSVL*

*TVLHQDWLNGKEYKCKVSNKGLPSSIEKTISKAKGQPREPQVYTLPPSQEEMTKNQVSLTCLVK*

*GFYPSDIAVEWESNGQPENNYKTTPPVLDSDGSFFLYSRLTVDKSRWQEGNVFSCSVMHEALH*

*NHYTQKSLSLSLG*

Extracellular domain of HLA-B*57:01, <u>peptide linker</u>, *IgG4 Fc*

SEQ ID NO 009         HLA-B58 IgG4 Fc fusion protein (synthetic construct)

GSHSMRYFYTAMSRPGRGEPRFIAVGYVDDTQFVRFDSDAASPRTEPRAPWIEQEGPEYWDG

ETRNMKASAQTYRENLRIALRYYNQSEAGSHIIQRMYGCDLGPDGRLLRGHDQSAYDGKDYIAL

NEDLSSWTAADTAAQITQRKWEAARVAEQLRAYLEGLCVEWLRRYLENGKETLQRADPPKTHV

THHPVSDHEATLRCWALGFYPAEITLTWQRDGEDQTQDTELVETRPAGDRTFQKWAAVVVPSG

EEQRYTCHVQHEGLPKPLTLRWEPSSQS*GGGGSGGGGS**ESKYGPPCPPCPAPEFLGGPSVFL*

*FPPKPKDTLMISRTPEVTCVVVDVSQEDPEVQFNWYVDGVEVHNAKTKPREEQFNSTYRVVSVL*

*TVLHQDWLNGKEYKCKVSNKGLPSSIEKTISKAKGQPREPQVYTLPPSQEEMTKNQVSLTCLVK*

*GFYPSDIAVEWESNGQPENNYKTTPPVLDSDGSFFLYSRLTVDKSRWQEGNVFSCSVMHEALH*

*NHYTQKSLSLSLG*

Extracellular domain of HLA-B*58:01, peptide linker, *IgG4 Fc*

SEQ ID NO 010　　HLA-C08 IgG4 Fc fusion protein (synthetic construct)

CSHSMRYFYTAVSRPGRGEPRFIAVGYVDDTQFVQFDSDAASPRGEPRAPWVEQEGPEYWDR

ETQKYKRQAQTDRVSLRNLRGYYNQSEAGSHTLQRMYGCDLGPDGRLLRGYNQFAYDGKDYI

ALNEDLRSWTAADKAAQITQRKWEAAREAEQRRAYLEGTCVEWLRRYLENGKKTLQRAEHPKT

HVTHHPVSDHEATLRCWALGFYPAEITLTWQRDGEDQTQDTELVETRPAGDGTFQKWAAVVVP

SGEEQRYTCHVQHEGLPEPLTLRWGPSSQP*GGGGSGGGGS**ESKYGPPCPPCPAPEFLGGPS*

*VFLFPPKPKDTLMISRTPEVTCVVVDVSQEDPEVQFNWYVDGVEVHNAKTKPREEQFNSTYRVV*

*SVLTVLHQDWLNGKEYKCKVSNKGLPSSIEKTISKAKGQPREPQVYTLPPSQEEMTKNQVSLTC*

*LVKGFYPSDIAVEWESNGQPENNYKTTPPVLDSDGSFFLYSRLTVDKSRWQEGNVFSCSVMHE*

*ALHNHYTQKSLSLSLG*

Extracellular domain of HLA-Cw0802, peptide linker, *IgG4 Fc*

SEQ ID NO 011　　HLA-A*30:01 E46A (synthetic construct)

(continued)

GSHSMRYFSTSVSRPGSGEPRFIAVGYVDDTQFVRFDSDAASQRMAPRAPWIEQERPEYWDQ

ETRNVKAQSQTDRVDLGTLRGYYNQSEAGSHTIQIMYGCDVGSDGRFLRGYEQHAYDGKDYIA

LNEDLRSWTAADMAAQITQRKWEAARWAEQLRAYLEGTCVEWLRRYLENGKETLQRTDPPKT

HMTHHPISDHEATLRCWALGFYPAEITLTWQRDGEDQTQDTELVETRPAGDGTFQKWAAVVVP

SGEEQRYTCHVQHEGLPKPLTLRWELSSQPGGGGSGGGGSESKYGPPCPPCPAPEFLGGPSV

FLFPPKPKDTLMISRTPEVTCVVVDVSQEDPEVQFNWYVDGVEVHNAKTKPREEQFNSTYRVVS

VLTVLHQDWLNGKEYKCKVSNKGLPSSIEKTISKAKGQPREPQVYTLPPSQEEMTKNQVSLTCL

VKGFYPSDIAVEWESNGQPENNYKTTPPVLDSDGSFFLYSRLTVDKSRWQEGNVFSCSVMHEA

LHNHYTQKSLSLSLG

SEQ ID NO 012          HLA-A*30:01 I97R (synthetic construct)

GSHSMRYFSTSVSRPGSGEPRFIAVGYVDDTQFVRFDSDAASQRMEPRAPWIEQERPEYWDQ

ETRNVKAQSQTDRVDLGTLRGYYNQSEAGSHTIQRMYGCDVGSDGRFLRGYEQHAYDGKDYIA

LNEDLRSWTAADMAAQITQRKWEAARWAEQLRAYLEGTCVEWLRRYLENGKETLQRTDPPKT

HMTHHPISDHEATLRCWALGFYPAEITLTWQRDGEDQTQDTELVETRPAGDGTFQKWAAVVVP

SGEEQRYTCHVQHEGLPKPLTLRWELSSQPGGGGSGGGGSESKYGPPCPPCPAPEFLGGPSV

FLFPPKPKDTLMISRTPEVTCVVVDVSQEDPEVQFNWYVDGVEVHNAKTKPREEQFNSTYRVVS

VLTVLHQDWLNGKEYKCKVSNKGLPSSIEKTISKAKGQPREPQVYTLPPSQEEMTKNQVSLTCL

VKGFYPSDIAVEWESNGQPENNYKTTPPVLDSDGSFFLYSRLTVDKSRWQEGNVFSCSVMHEA

LHNHYTQKSLSLSLG

SEQ ID NO 013          HLA-A*30:01 E46A / I97R (synthetic construct)

GSHSMRYFSTSVSRPGSGEPRFIAVGYVDDTQFVRFDSDAASQRMAPRAPWIEQERPEYWDQ

ETRNVKAQSQTDRVDLGTLRGYYNQSEAGSHTIQRMYGCDVGSDGRFLRGYEQHAYDGKDYIA

LNEDLRSWTAADMAAQITQRKWEAARWAEQLRAYLEGTCVEWLRRYLENGKETLQRTDPPKT

HMTHHPISDHEATLRCWALGFYPAEITLTWQRDGEDQTQDTELVETRPAGDGTFQKWAAVVVP

SGEEQRYTCHVQHEGLPKPLTLRWELSSQPGGGGSGGGGSESKYGPPCPPCPAPEFLGGPSV

FLFPPKPKDTLMISRTPEVTCVVVDVSQEDPEVQFNWYVDGVEVHNAKTKPREEQFNSTYRVVS

VLTVLHQDWLNGKEYKCKVSNKGLPSSIEKTISKAKGQPREPQVYTLPPSQEEMTKNQVSLTCL

VKGFYPSDIAVEWESNGQPENNYKTTPPVLDSDGSFFLYSRLTVDKSRWQEGNVFSCSVMHEA

LHNHYTQKSLSLSLG

SEQ ID NO 014    HLA-B*5701 A46E (synthetic construct)

GSHSMRYFYTAMSRPGRGEPRFIAVGYVDDTQFVRFDSDAASPRMEPRAPWIEQEGPEYWDG

ETRNMKASAQTYRENLRIALRYYNQSEAGSHIIQVMYGCDVGPDGRLLRGHDQSAYDGKDYIAL

NEDLSSWTAADTAAQITQRKWEAARVAEQLRAYLEGLCVEWLRRYLENGKETLQRADPPKTHV

THHPISDHEATLRCWALGFYPAEITLTWQRDGEDQTQDTELVETRPAGDRTFQKWAAVVVPSG

EEQRYTCHVQHEGLPKPLTLRWEPSSQSGGGGSGGGGSESKYGPPCPPCPAPEFLGGPSVFL

FPPKPKDTLMISRTPEVTCVVVDVSQEDPEVQFNWYVDGVEVHNAKTKPREEQFNSTYRVVSVL

TVLHQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEALH

NHYTQKSLSLSLG

SEQ ID NO 015    HLA-B*5701 V97R (synthetic construct)

GSHSMRYFYTAMSRPGRGEPRFIAVGYVDDTQFVRFDSDAASPRMAPRAPWIEQEGPEYWDG

ETRNMKASAQTYRENLRIALRYYNQSEAGSHIIQRMYGCDVGPDGRLLRGHDQSAYDGKDYIAL

NEDLSSWTAADTAAQITQRKWEAARVAEQLRAYLEGLCVEWLRRYLENGKETLQRADPPKTHV

THHPISDHEATLRCWALGFYPAEITLTWQRDGEDQTQDTELVETRPAGDRTFQKWAAVVVPSG

EEQRYTCHVQHEGLPKPLTLRWEPSSQSGGGGSGGGGSESKYGPPCPPCPAPEFLGGPSVFL

FPPKPKDTLMISRTPEVTCVVVDVSQEDPEVQFNWYVDGVEVHNAKTKPREEQFNSTYRVVSVL

TVLHQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEALH

NHYTQKSLSLSLG

SEQ ID NO 016          HLA-B*58:01 E46A (synthetic construct)

GSHSMRYFYTAMSRPGRGEPRFIAVGYVDDTQFVRFDSDAASPRTAPRAPWIEQEGPEYWDG

ETRNMKASAQTYRENLRIALRYYNQSEAGSHIIQRMYGCDLGPDGRLLRGHDQSAYDGKDYIAL

NEDLSSWTAADTAAQITQRKWEAARVAEQLRAYLEGLCVEWLRRYLENGKETLQRADPPKTHV

THHPVSDHEATLRCWALGFYPAEITLTWQRDGEDQTQDTELVETRPAGDRTFQKWAAVVVPSG

EEQRYTCHVQHEGLPKPLTLRWEPSSQSGGGGSGGGGSESKYGPPCPPCPAPEFLGGPSVFL

FPPKPKDTLMISRTPEVTCVVVDVSQEDPEVQFNWYVDGVEVHNAKTKPREEQFNSTYRVVSVL

TVLHQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEALH

NHYTQKSLSLSLG

SEQ ID NO 017          HLA-B*58:01 R97V (synthetic construct)

EP 4 380 968 B1

(continued)

GSHSMRYFYTAMSRPGRGEPRFIAVGYVDDTQFVRFDSDAASPRTEPRAPWIEQEGPEYWDG

ETRNMKASAQTYRENLRIALRYYNQSEAGSHIIQVMYGCDLGPDGRLLRGHDQSAYDGKDYIAL

NEDLSSWTAADTAAQITQRKWEAARVAEQLRAYLEGLCVEWLRRYLENGKETLQRADPPKTHV

THHPVSDHEATLRCWALGFYPAEITLTWQRDGEDQTQDTELVETRPAGDRTFQKWAAVVVPSG

EEQRYTCHVQHEGLPKPLTLRWEPSSQSGGGGSGGGGSESKYGPPCPPCPAPEFLGGPSVFL

FPPKPKDTLMISRTPEVTCVVVDVSQEDPEVQFNWYVDGVEVHNAKTKPREEQFNSTYRVVSVL

TVLHQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEALH

NHYTQKSLSLSLG

SEQ ID NO 018        HLA-B*58:01 E46A, R97V (synthetic construct)

GSHSMRYFYTAMSRPGRGEPRFIAVGYVDDTQFVRFDSDAASPRTAPRAPWIEQEGPEYWDG

ETRNMKASAQTYRENLRIALRYYNQSEAGSHIIQVMYGCDLGPDGRLLRGHDQSAYDGKDYIAL

NEDLSSWTAADTAAQITQRKWEAARVAEQLRAYLEGLCVEWLRRYLENGKETLQRADPPKTHV

THHPVSDHEATLRCWALGFYPAEITLTWQRDGEDQTQDTELVETRPAGDRTFQKWAAVVVPSG

EEQRYTCHVQHEGLPKPLTLRWEPSSQSGGGGSGGGGSESKYGPPCPPCPAPEFLGGPSVFL

FPPKPKDTLMISRTPEVTCVVVDVSQEDPEVQFNWYVDGVEVHNAKTKPREEQFNSTYRVVSVL

TVLHQDWLNGKEYKCKVSNKGLPSSIEKTISKAKGQPREPQVYTLPPSQEEMTKNQVSLTCLVK

GFYPSDIAVEWESNGQPENNYKTTPPVLDSDGSFFLYSRLTVDKSRWQEGNVFSCSVMHEALH

NHYTQKSLSLSLG

SEQ ID NO 019        HLA-Cw0802 E46A (synthetic construct)

CSHSMRYFYTAVSRPGRGEPRFIAVGYVDDTQFVQFDSDAASPRGAPRAPWWEQEGPEYWDR

ETQKYKRQAQTDRVSLRNLRGYYNQSEAGSHTLQRMYGCDLGPDGRLLRGYNQFAYDGKDYI

ALNEDLRSWTAADKAAQITQRKWEAAREAEQRRAYLEGTCVEWLRRYLENGKKTLQRAEHPKT

HVTHHPVSDHEATLRCWALGFYPAEITLTWQRDGEDQTQDTELVETRPAGDGTFQKWAAVVVP

SGEEQRYTCHVQHEGLPEPLTLRWGPSSQPGGGGSGGGGSESKYGPPCPPCPAPEFLGGPS

VFLFPPKPKDTLMISRTPEVTCVVVDVSQEDPEVQFNWYVDGVEVHNAKTKPREEQFNSTYRVV

SVLTVLHQDWLNGKEYKCKVSNKGLPSSIEKTISKAKGQPREPQVYTLPPSQEEMTKNQVSLTC

LVKGFYPSDIAVEWESNGQPENNYKTTPPVLDSDGSFFLYSRLTVDKSRWQEGNVFSCSVMHE

ALHNHYTQKSLSLSLG

SEQ ID NO 020        HLA-Cw0802 R97V (synthetic construct)

CSHSMRYFYTAVSRPGRGEPRFIAVGYVDDTQFVQFDSDAASPRGEPRAPWWEQEGPEYWDR

ETQKYKRQAQTDRVSLRNLRGYYNQSEAGSHTLQVMYGCDLGPDGRLLRGYNQFAYDGKDYI

ALNEDLRSWTAADKAAQITQRKWEAAREAEQRRAYLEGTCVEWLRRYLENGKKTLQRAEHPKT

HVTHHPVSDHEATLRCWALGFYPAEITLTWQRDGEDQTQDTELVETRPAGDGTFQKWAAVVVP

SGEEQRYTCHVQHEGLPEPLTLRWGPSSQPGGGGSGGGGSESKYGPPCPPCPAPEFLGGPS

VFLFPPKPKDTLMISRTPEVTCVVVDVSQEDPEVQFNWYVDGVEVHNAKTKPREEQFNSTYRVV

SVLTVLHQDWLNGKEYKCKVSNKGLPSSIEKTISKAKGQPREPQVYTLPPSQEEMTKNQVSLTC

LVKGFYPSDIAVEWESNGQPENNYKTTPPVLDSDGSFFLYSRLTVDKSRWQEGNVFSCSVMHE

ALHNHYTQKSLSLSLG

SEQ ID NO 021        HLA-Cw0802 E46A, R97V (synthetic construct)

CSHSMRYFYTAVSRPGRGEPRFIAVGYVDDTQFVQFDSDAASPRGAPRAPWVEQEGPEYWDR

ETQKYKRQAQTDRVSLRNLRGYYNQSEAGSHTLQVMYGCDLGPDGRLLRGYNQFAYDGKDYI

ALNEDLRSWTAADKAAQITQRKWEAAREAEQRRAYLEGTCVEWLRRYLENGKKTLQRAEHPKT

HVTHHPVSDHEATLRCWALGFYPAEITLTWQRDGEDQTQDTELVETRPAGDGTFQKWAAVVVP

SGEEQRYTCHVQHEGLPEPLTLRWGPSSQPGGGGSGGGGSESKYGPPCPPCPAPEFLGGPS

VFLFPPKPKDTLMISRTPEVTCVVVDVSQEDPEVQFNWYVDGVEVHNAKTKPREEQFNSTYRVV

SVLTVLHQDWLNGKEYKCKVSNKGLPSSIEKTISKAKGQPREPQVYTLPPSQEEMTKNQVSLTC

LVKGFYPSDIAVEWESNGQPENNYKTTPPVLDSDGSFFLYSRLTVDKSRWQEGNVFSCSVMHE

ALHNHYTQKSLSLSLG

**Claims**

1. A pharmaceutical composition for use in the treatment of cancer, said composition comprising:

   a. an HLA fusion protein comprising:

      i. a human leukocyte antigen (HLA) heavy chain polypeptide selected from:

         • an extracellular domain of an HLA heavy chain, particularly an HLA heavy chain selected from HLA-B57, HLA-C08, HLA-A25, HLA-B58, HLA-B27, HLA-A30, HLA-B53, or HLA-C12; or
         • a variant of said extracellular domain of an HLA heavy chain, wherein said variant is **characterized by** a sequence similarity of at least ($\geq$) 95%, and a similar biological activity, in comparison to the respective extracellular domain of the HLA heavy chain;

      ii. an immunoglobulin crystallizable fragment (Ig Fc) polypeptide; and

   b. a beta 2 microglobulin (B2m) polypeptide.

2. The pharmaceutical composition for use according to claim 1, wherein the HLA fusion protein is non-covalently associated with the B2m polypeptide.

3. The pharmaceutical composition for use according to claim 1 or 2, wherein the HLA fusion protein is non-covalently associated with the B2m polypeptide at a ratio of between 3:5 to 7:5.

4. The pharmaceutical composition for use according to any one of the claims 1 to 3, wherein the HLA heavy chain polypeptide is a variant of the extracellular domain of HLA-B57, and wherein HLA heavy chain polypeptide is **characterized by** an E at position 46, and an R at position 97.

5. The pharmaceutical composition for use according to any one of the claims 1 to 4, wherein the HLA heavy chain polypeptide comprises, or essentially consists of, the sequence SEQ ID NO 001.

6. The pharmaceutical composition for use according to any one of the claims 1 to 5, wherein the HLA fusion protein comprises:

   a. the HLA heavy chain polypeptide as specified in any one of the claims 1 to 5; and
   b. an IgG Fc polypeptide; and optionally
   c. a peptide linker connecting the HLA heavy chain polypeptide to the IgG Fc polypeptide;

   and wherein optionally, the HLA fusion protein further comprises:
   d. a secretory signal.

7. The pharmaceutical composition for use according to any one of the claims 1 to 6, wherein the HLA heavy chain polypeptide is positioned N-terminal relative to the IgG Fc polypeptide.

8. The pharmaceutical composition for use according to any one of the claims 1 to 7, wherein the HLA fusion protein comprises, or essentially consists of, the sequence designated SEQ ID NO 005.

9. The pharmaceutical composition for use according to any one of the claims 1 to 8, wherein the HLA fusion protein is in the form of a dimer, said dimer comprising, or essentially consisting of a first HLA monomer and a second HLA monomer;

   - wherein the first HLA monomer essentially consists of a first HLA fusion protein as specified in any one of the claims 1 to 8, and a first B2m polypeptide; and
   - wherein the second HLA monomer essentially consists of a second HLA fusion protein as specified in any one of the claims 1 to 8, and a second B2m polypeptide.

10. The pharmaceutical composition for use according to any one of the claims 1 to 9, wherein the HLA fusion protein is not associated with a peptide epitope.

**11.** The pharmaceutical composition for use according to any one of the claims 1 to 10, wherein the pharmaceutical composition is administered prior to, in combination with, or subsequent to a checkpoint inhibitory agent, and wherein said checkpoint inhibitory agent is an antibody capable of binding to one of CTLA-4, PD-1, PD-L1, or PD-L2 with a dissociation constant of $10^{-7}$ mol/L or lower.

**12.** A checkpoint inhibitory agent for use in the treatment of cancer, wherein the checkpoint inhibitory agent is administered prior to, in combination with, or subsequent to the pharmaceutical composition for use according to any one of the claims 1 to 11, and wherein said checkpoint inhibitory agent is an antibody capable of binding to one of CTLA-4, PD-1, PD-L1, or PD-L2 with a dissociation constant of $10^{-7}$ mol/L or lower.

**13.** The pharmaceutical composition for use according to any claim 11, or the checkpoint inhibitory agent for use according to claim 12, wherein said checkpoint inhibitory agent is provided in a dosage form suitable for systemic delivery.

**14.** A pharmaceutical composition for use according to any one of the claims 1 to 11, or 13, wherein the cancer is

    a. a blood cell-derived cancer, or
    b. a solid tumor.

**15.** The checkpoint inhibitory agent for use according to any one of the claims 12 or 13, wherein the malignant neoplastic disease is selected from colon cancer, breast cancer, pancreatic cancer, or melanoma.

**Patentansprüche**

**1.** Pharmazeutische Zusammensetzung zur Verwendung bei der Behandlung von Krebs, wobei die Zusammensetzung umfasst:

    a. ein HLA-Fusionsprotein, das umfasst:

        i. ein Schwerketten-Polypeptid eines humanen Leukozytenantigens (HLA), ausgewählt aus:

            · einer extrazellulären Domäne einer HLA-Schwerkette, insbesondere einer HLA-Schwerkette, ausgewählt aus HLA-B57, HLA-C08, HLA-A25, HLA-B58, HLA-B27, HLA-A30, HLA-B53 oder HLA-C12; oder
            · einer Variante der extrazellulären Domäne einer HLA-Schwerkette, wobei die Variante durch eine Sequenzähnlichkeit von mindestens ($\geq$) 95 % und eine ähnliche biologische Aktivität im Vergleich zur jeweiligen extrazellulären Domäne der HLA-Schwerkette gekennzeichnet ist;

        ii. ein (Ig Fc)-Polypeptid der fragment-kristallisierbaren Region (Fc) eines Immunglobulins (Ig); und

    b. ein Beta-2-Mikroglobulin (B2m)-Polypeptid.

**2.** Die pharmazeutische Zusammensetzung zur Verwendung nach Anspruch 1, wobei das HLA-Fusionsprotein nicht-kovalent mit dem B2m-Polypeptid assoziiert ist.

**3.** Die pharmazeutische Zusammensetzung zur Verwendung nach Anspruch 1 oder 2, wobei das HLA-Fusionsprotein nicht-kovalent mit dem B2m-Polypeptid in einem Verhältnis von 3:5 bis 7:5 assoziiert ist.

**4.** Die pharmazeutische Zusammensetzung zur Verwendung nach einem der Ansprüche 1 bis 3, wobei das HLA-Schwerketten-Polypeptid eine Variante der extrazellulären Domäne von HLA-B57 ist und wobei das HLA-Schwerketten-Polypeptid durch ein E an Position 46 und ein R an Position 97 gekennzeichnet ist.

**5.** Die pharmazeutische Zusammensetzung zur Verwendung nach einem der Ansprüche 1 bis 4, wobei das HLA-Schwerketten-Polypeptid die Sequenz SEQ ID NO 001 umfasst oder im Wesentlichen aus dieser besteht.

**6.** Die pharmazeutische Zusammensetzung zur Verwendung nach einem der Ansprüche 1 bis 5, wobei das HLA-Fusionsprotein umfasst:

    a. das HLA-Schwerketten-Polypeptid, wie in einem der Ansprüche 1 bis 5 angegeben; und
    b. ein IgG-Fc-Polypeptid; und optional
    c. einen Peptidlinker, der das HLA-Schwerketten-Polypeptid mit dem IgG-Fc-Polypeptid verbindet;

und wobei das HLA-Fusionsprotein optional ferner umfasst:
d. ein Sekretionssignal

**7.** Die pharmazeutische Zusammensetzung zur Verwendung nach einem der Ansprüche 1 bis 6, wobei das HLA-Schwerketten-Polypeptid N-terminal relativ zum IgG-Fc-Polypeptid positioniert ist.

**8.** Die pharmazeutische Zusammensetzung zur Verwendung nach einem der Ansprüche 1 bis 7, wobei das HLA-Fusionsprotein die Sequenz SEQ ID NO 005 umfasst oder im Wesentlichen daraus besteht.

**9.** Die pharmazeutische Zusammensetzung zur Verwendung nach einem der Ansprüche 1 bis 8, wobei das HLA-Fusionsprotein in Form eines Dimers vorliegt, wobei das Dimer ein erstes HLA-Monomer und ein zweites HLA-Monomer umfasst oder im Wesentlichen daraus besteht;

    - wobei das erste HLA-Monomer im Wesentlichen aus einem ersten HLA-Fusionsprotein, wie in einem der Ansprüche 1 bis 8 angegeben, und einem ersten B2m-Polypeptid besteht; und
    - wobei das zweite HLA-Monomer im Wesentlichen aus einem zweiten HLA-Fusionsprotein, wie in einem der Ansprüche 1 bis 8 angegeben, und einem zweiten B2m-Polypeptid besteht.

**10.** Die pharmazeutische Zusammensetzung zur Verwendung nach einem der Ansprüche 1 bis 9, wobei das HLA-Fusionsprotein nicht mit einem Peptidepitop assoziiert ist.

**11.** Die pharmazeutische Zusammensetzung zur Verwendung nach einem der Ansprüche 1 bis 10, wobei die pharmazeutische Zusammensetzung vor, in Kombination mit oder nach einem Checkpoint-Inhibitor verabreicht wird und wobei der Checkpoint-Inhibitor ein Antikörper ist, der in der Lage ist, an CTLA-4, PD-1, PD-L1 oder PD-L2 mit einer Dissoziationskonstante von $10^{-7}$ mol/l oder weniger zu binden.

**12.** Ein Checkpoint-Inhibitor zur Verwendung bei der Behandlung von Krebs, wobei der Checkpoint-Inhibitor vor, in Kombination mit oder nach der pharmazeutischen Zusammensetzung zur Verwendung nach einem der Ansprüche 1 bis 11 verabreicht wird und wobei der Checkpoint-Inhibitor ein Antikörper ist, der an eines von CTLA-4, PD-1, PD-L1 oder PD-L2 mit einer Dissoziationskonstante von $10^{-7}$ mol/l oder weniger binden kann.

**13.** Die pharmazeutische Zusammensetzung zur Verwendung nach einem der Ansprüche 11 oder der Checkpoint-Inhibitor zur Verwendung nach Anspruch 12, wobei der Checkpoint-Inhibitor in einer zur systemischen Verabreichung geeigneten Darreichungsform bereitgestellt ist.

**14.** Die pharmazeutische Zusammensetzung zur Verwendung nach einem der Ansprüche 1 bis 11 oder 13, wobei der Krebs

    a. ein aus Blutzellen stammender Krebs oder
    b. ein solider Tumor ist.

**15.** Der Checkpoint-Inhibitor zur Verwendung nach einem der Ansprüche 12 oder 13, wobei die maligne neoplastische Erkrankung aus Darmkrebs, Brustkrebs, Bauchspeicheldrüsenkrebs oder Melanom ausgewählt ist.

**Revendications**

**1.** Composition pharmaceutique destinée à être utilisée dans le traitement du cancer, ladite composition comprenant :

    a. une protéine de fusion de HLA comprenant :

i. un polypeptide à chaîne lourde d'antigène leucocytaire humain (HLA) sélectionné parmi :

• un domaine extracellulaire d'une chaîne lourde de HLA, notamment une chaîne lourde de HLA sélectionnée parmi HLA-B57, HLA-C08, HLA-A25, HLA-B58, HLA-B27, HLA-A30, HLA-B53 ou HLA-C12 ; ou
• une variante dudit domaine extracellulaire d'une chaîne lourde de HLA, dans laquelle ladite variante est **caractérisée par** une similarité de séquence d'au moins ($\geq$) 95 %, et une activité biologique similaire, en comparaison au domaine extracellulaire de la chaîne lourde de HLA ;

ii. un polypeptide à fragment cristallisable d'immunoglobuline (Ig Fc) ; et

b. un polypeptide de bêta 2 microglobuline (B2m).

**2.** Composition pharmaceutique destinée à être utilisée selon la revendication 1, dans laquelle la protéine de fusion de HLA est associée de manière non covalente au polypeptide B2m.

**3.** Composition pharmaceutique destinée à être utilisée selon la revendication 1 ou 2, dans laquelle la protéine de fusion de HLA est associée de manière non covalente au polypeptide B2m à un rapport compris entre 3/5 et 7/5.

**4.** Composition pharmaceutique destinée à être utilisée selon l'une quelconque des revendications 1 à 3, dans laquelle le polypeptide à chaîne lourde de HLA est une variante du domaine extracellulaire de HLA-B57, et dans laquelle le polypeptide à chaîne lourde de HLA est **caractérisé par** un E à la position 46 et un R à la position 97.

**5.** Composition pharmaceutique destinée à être utilisée selon l'une quelconque des revendications 1 à 4, dans laquelle le polypeptide à chaîne lourde de HLA comprend, ou est essentiellement constitué de, la séquence SEQ ID N° 001.

**6.** Composition pharmaceutique destinée à être utilisée selon l'une quelconque des revendications 1 à 5, dans laquelle la protéine de fusion de HLA comprend :

a. le polypeptide à chaîne lourde de HLA tel que spécifié dans l'une quelconque des revendications 1 à 5 ; et
b. un polypeptide IgG Fc ; et en option
c. un liant peptidique connectant le polypeptide à chaîne lourde de HLA au polypeptide IgG Fc ;

et dans laquelle en option, la protéine de fusion de HLA comprend en outre :
d. un signal sécrétoire.

**7.** Composition pharmaceutique destinée à être utilisée selon l'une quelconque des revendications 1 à 6, dans laquelle le polypeptide à chaîne lourde de HLA est positionné à la terminaison N par rapport au polypeptide IgG Fc.

**8.** Composition pharmaceutique destinée à être utilisée selon l'une quelconque des revendications 1 à 7, dans laquelle la protéine de fusion de HLA comprend, ou est essentiellement constituée de, la séquence désignée par SEQ ID N° 005.

**9.** Composition pharmaceutique destinée à être utilisée selon l'une quelconque des revendications 1 à 8, dans laquelle la protéine de fusion de HLA est sous la forme d'un dimère, ledit dimère comprenant, ou étant essentiellement constitué d'un premier monomère de HLA et d'un second monomère de HLA ;

- dans laquelle le premier monomère de HLA est essentiellement constitué d'une première protéine de fusion de HLA telle que spécifiée dans l'une quelconque des revendications 1 à 8, et d'un premier polypeptide B2m ; et
- dans laquelle le second monomère de HLA est essentiellement constitué d'une seconde protéine de fusion de HLA telle que spécifiée dans l'une quelconque des revendications 1 à 8, et d'un second polypeptide B2m.

**10.** Composition pharmaceutique destinée à être utilisée selon l'une quelconque des revendications 1 à 9, dans laquelle la protéine de fusion de HLA n'est pas associée à un épitope peptidique.

**11.** Composition pharmaceutique destinée à être utilisée selon l'une quelconque des revendications 1 à 10, dans laquelle la composition pharmaceutique est administrée avant, en combinaison à, ou de manière subséquente à un agent inhibiteur de point de contrôle, et dans laquelle ledit agent inhibiteur de point de contrôle est un anticorps capable de se lier à un de CTLA-4, PD-1, PD-L1 ou PD-L2 avec une constante de dissociation de $10^{-7}$ mol/l ou inférieure.

12. Agent inhibiteur de point de contrôle destiné à être utilisé dans le traitement du cancer, dans lequel l'agent inhibiteur de point de contrôle est administré avant, en combinaison à, ou de manière subséquente à la composition pharmaceutique destinée à être utilisée selon l'une quelconque des revendications 1 à 11, et dans lequel ledit agent inhibiteur de point de contrôle est un anticorps capable de se lier à un de CTLA-4, PD-1, PD-L1 ou PD-L2 avec une constante de dissociation de $10^{-7}$ mol/l ou inférieure.

13. Composition pharmaceutique destinée à être utilisée selon l'une quelconque de la revendication 11, ou agent inhibiteur de point de contrôle destiné à être utilisé selon la revendication 12, dans laquelle/lequel ledit agent inhibiteur de point de contrôle est fourni en une forme de dosage convenant à l'administration systémique.

14. Composition pharmaceutique destinée à être utilisée selon l'une quelconque des revendications 1 à 11, ou 13, dans laquelle le cancer est

    a. un cancer dérivé de cellule sanguine, ou
    b. une tumeur solide.

15. Agent inhibiteur de point de contrôle destiné à être utilisé selon l'une quelconque des revendications 12 ou 13, dans lequel la maladie néoplasique maligne est sélectionnée parmi le cancer du côlon, le cancer du sein, le cancer pancréatique ou le mélanome.

Fig.1

A

B

Fig. 1 (continued)

| iosH2 Variant | Ratio iosH2.β2m | Process duration | Cell viability (%) | Titer (μg/mL) |
|---|---|---|---|---|
| HLA-B57(A46E/V97R).β2m | 1:0 | 14 | 49 | 32 |
| HLA-B57(A46E/V97R).β2m | 1:1 | 14 | 77 | 1379 |
| HLA-B57(A46E/V97R).β2m | 1:2 | 14 | 75 | 1214 |
| HLA-B57(A46E/V97R).β2m | 1:4 | 14 | 69 | 997 |
| HLA-B57.β2m | 1:0 | 14 | 39 | 19 |
| HLA-B57.β2m | 1:1 | 14 | 24 | 83 |
| HLA-B57.β2m | 1:2 | 14 | 27 | 114 |
| HLA-B57.β2m | 1:4 | 14 | 29 | 95 |

HLA-B57$^{(A46E/V97R)}$.β2m 1:0
HLA-B57$^{(A46E/V97R)}$.β2m 1:1
HLA-B57$^{(A46E/V97R)}$.β2m 1:2
HLA-B57$^{(A46E/V97R)}$.β2m 4:1
HLA-B57.β2m 1:0
HLA-B57.β2m 1:1
HLA-B57.β2m 1:2
HLA-B57.β2m 4:1

C

Viability %

% of viable cells

Process Duration (Days)

D

Titer

Titer (μg/ml)

Process Duration (Days)

Fig. 2

Fig. 2 (continued)

B

HLA-B57$^{(A46E/V97R)}$

A **Supernatant**
(transient transfection CHO)

B **Affinity purification**

β2m removal
with low pH

D SEC purification of
HLA-B57$^{(A46E/V97R)}$

C SEC purification of
HLA-B57$^{(A46E/V97R)}$.β2m

| Step | HLA-B57 titers (%) | HLA-B57$^{(A46E/V97R)}$ titers (%) |
|---|---|---|
| CHO supernatant | 20 mg (100%) | 96 mg (100%) |
| Affinity purification | 14 mg (70%) | 67 mg (70%) |
| SEC purified B2m complex | 4.4 mg (22%) | 48 g (50%) |
| SEC purified B2m-free | 2 mg (10%) | 24 mg (25%) |

Fig. 2
(continued)

**C**

Fig 3.

A

HLA-B57

HLA-B57$^{(A46E/V97R)}$

HLA-B57$^{(A46E/V97R)}$.β2m

log10(nM)

Fig. 3 (continued)

| | HLA-B57[(A46E/V97R)] | HLA-B57[(A46E/V97R)].B2m |
|---|---|---|
| **Kd (affinity)** | 20.3 nM | 2.3 nM |
| **Kon** | $7.6 \times 10^4$ | $6.47 \times 10^4$ |
| **Koff** | $1.75 \times 10^{-3}$ | $1.53 \times 10^{-4}$ |

Fig. 4

H69 lung cancer

A549 lung cancer

Fig. 4 (continued)

BT474 breast Cancer

Daudi lymphoma

Fig. 4 (continued)

RPMI-8226 Myeloma

Jurkat Leukemia

Fig. 5

A

First death B57.B2m

First death B57.

First death Ctrl.

First death IgG4

Ctrl. untreated
Isotype IgG4
B57.
B57.B2m

Fig. 5 (continued)

B

Ctrl. untreated
IgG4
B57
B57.B2m

Ctrl. vs. B57        p<0.16
IgG4 vs. B57        p<0.32

Ctrl. vs. B57.B2m   p<0.046
IgG4 vs. B57.B2m  p<0.15

EP 4 380 968 B1

Fig. 6

Fig. 7

A

EP 4 380 968 B1

Fig. 7 (continued)

Fig. 8

**A** LILRB1

**B** LILRB2

Fig. 9

A

Lung (H69)

Leukemia (Jurkat)

Fig. 9 (continued)          B

Pancreatic cancer (Mia Paca2)

Lung (H460)

Myeloma (RPMI8226)

Lymphoma (Daudi)

IgG4

IosH2

TTI 621

TTI 622

Fig. 10

A

Single agent therapy

C

Fig. 10 (continued)

Fig. 11

A                          Human primary NK cells

Sorted KIR3DL1+ NK cells

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 21190004
- EP 21190005
- EP 21207324
- WO 2017153438 A1
- WO 2016124661 A1
- WO 2018029284 A1
- US 20180298096 A1

**Non-patent literature cited in the description**

- **AROSA et al.** *Trends in Immunology*, March 2007, vol. 28 (3), 115-23
- **SAMBROOK et al.** Molecular Cloning: A Laboratory Manual. Cold Spring Harbor Laboratory Press, 2012
- **AUSUBEL et al.** Short Protocols in Molecular Biology. John Wiley & Sons, Inc., 2002
- **STRYER**. Biochemistry, 21
- **SMITH** ; **WATERMAN**. *Adv. Appl. Math.*, 1981, vol. 2, 482
- **NEEDLEMAN** ; **WUNSCH**. *J. Mol. Biol.*, 1970, vol. 48, 443
- **PEARSON** ; **LIPMAN**. *Proc. Nat. Acad. Sci.*, 1988, vol. 85, 2444
- **ALTSCHUL et al.** *J. Mol. Biol.*, 1990, vol. 215, 403-410
- *Remington: the Science and Practice of Pharmacy*, ISBN 0857110624
- **L. LACHMAN et al.** The Theory and Practice of Industrial Pharmacy. 2013
- *CHEMICAL ABSTRACTS*, 477202-00-9
- *CHEMICAL ABSTRACTS*, 745013-59-6
- *CHEMICAL ABSTRACTS*, 946414-94-4
- *CHEMICAL ABSTRACTS*, 1036730-42-3
- *CHEMICAL ABSTRACTS*, 1380723-44-3
- *CHEMICAL ABSTRACTS*, 1537032-82-8
- *CHEMICAL ABSTRACTS*, 1428935-60-7
- *CHEMICAL ABSTRACTS*, 1801342-60-8
- *CHEMICAL ABSTRACTS*, 1374853-91-4
- **LIN G**. *PLos One*, 2017, vol. 12 (10), e0187626